# FASCICULE DE BREVET EUROPEEN

(11) **EP 2 897 705 B1**
(45) Date de publication et mention de la délivrance du brevet: **09.09.2020**
(21) Numéro de dépôt: 13774757.2
(22) Date de dépôt: 18.09.2013
(51) Int. Cl.: G01N 33/18, G01N 1/40, G01N 33/28, B01D 17/02, E21B 43/34

(54) **INSTALLATION DE TRAITEMENT D'UN FLUIDE MULTIPHASIQUE ET PROCEDE DE CARACTERISATION EN LIGNE DUDIT FLUIDE**
ANLAGE ZUR VERARBEITUNG EINES MEHRPHASIGEN FLUIDS UND VERFAHREN ZUR ONLINE-CHARAKTERISIERUNG DIESES FLUIDS
FACILITY FOR PROCESSING A MULTIPHASE FLUID AND METHOD FOR CHARACTERIZING SAID FLUID ONLINE

(30) Priorité: 18.09.2012 FR 1258761
(43) Date de publication de la demande: 29.07.2015
(73) Titulaire: Wintech Global, 69003 Lyon (FR)
(72) Inventeur: SZYMKOWIAK, Bertrand, Jean, F-69005 Lyon (FR); LECOFFRE, Yves, Louis, Léon, Marie, F-38000 Grenoble (FR); MAJ, Guillaume, Daniel, Ghislain, F-38400 Saint Martin D'heres (FR)
(74) Mandataire: Weber, Jean-François
(86) Numéro de dépôt international: PCT/FR2013/052158
(87) Numéro de publication internationale: WO 2014/044973

(56) Documents cités:
- WO-A1-2006/085772
- DE-A1-102005 017 614
- US-A- 5 526 684
- US-A1- 2005 016 292
- US-A1- 2007 274 842
- US-A1- 2010 174 517

## Description

### DOMAINE TECHNIQUE

La présente invention se rapporte au domaine technique général du traitement des fluides multiphasiques, et concerne plus particulièrement une installation destinée à être utilisée dans un cadre industriel, par exemple dans le secteur de la production pétrolière, de l'épuration des eaux usées, de l'agroalimentaire, de la cosmétique ou de la pharmaceutique.

La présente invention concerne plus précisément une installation de traitement d'un fluide multiphasique comprenant au moins deux phases de densités respectives différentes, ladite installation comprenant un circuit au sein duquel est destiné à circuler ledit fluide.

La présente invention concerne également un procédé de caractérisation d'un fluide multiphasique comprenant au moins deux phases de densités respectives différentes, ledit fluide étant destiné à circuler au sein d'un circuit.

### TECHNIQUE ANTERIEURE

Les fluides multiphasiques sont formés de phases de densités respectives différentes. Travailler avec de tels fluides est courant au sein de nombreuses industries. Une phase peut être gazeuse, liquide ou solide. Au sens de l'invention, un liquide constitue une phase lorsque les différents éléments le composant ne peuvent pas être séparés par gravité. Ainsi, un mélange de liquides miscibles constitue une seule et même phase.

Dans certains procédés industriels, il s'avère nécessaire de séparer les différentes phases d'un même fluide, c'est-à-dire séparer un ou plusieurs composants au sein d'une même composition. Cela est notamment le cas dans les sites de production ou de traitement de fluides pétroliers qui ont notamment pour objectif la séparation de l'eau et du brut de pétrole présents dans le fluide recueilli à la sortie du puits connecté au gisement pétrolier.

Dans d'autres situations, il est simplement utile de connaître les caractéristiques de la séparation des différentes phases du fluide multiphasique sous l'effet de la gravité. Ces caractéristiques peuvent s'avérer utiles pour piloter de manière optimale l'installation industrielle au sein de laquelle le fluide circule. A titre d'exemple, on peut chercher à s'assurer que les différentes phases d'un fluide n'ont pas le temps de se séparer lors d'un procédé industriel comportant notamment une phase de stockage, ou de manière plus générale une phase de transfert, par exemple par écoulement dans une conduite reliant deux étapes successives d'un procédé industriel.

A titre illustratif et non limitatif, nous allons nous focaliser sur le cas où l'installation de traitement constitue un élément d'une unité de production pétrolière, ledit fluide comprenant du pétrole. Cet exemple constitue l'application préférentielle de l'invention. Une contamination aqueuse du pétrole peut avoir lieu lorsque le gisement est en contact avec une nappe phréatique. Il se forme dès lors une émulsion comportant notamment une phase aqueuse et une phase organique, des gouttelettes de l'une des phases étant présentes dans l'autre phase. Cette émulsion issue de l'extraction pétrolière comprend en outre généralement une phase gazeuse. Avant toute opération ultérieure, il est donc nécessaire de séparer les différentes phases pour recueillir le brut de pétrole (la phase organique liquide) et rejeter la phase aqueuse d'une part et la phase gazeuse d'autre part. Il est primordial que cette séparation soit particulièrement efficace car des dispositions réglementaires, destinées à protéger l'environnement, obligent les producteurs de pétrole à limiter les rejets de pétrole et donc à diminuer fortement la teneur de la phase organique au sein de la phase aqueuse destinée à être rejetée dans le milieu naturel. L'installation de séparation doit donc satisfaire à des exigences qui peuvent être sévères en ce qui concerne la qualité des fluides en sortie.

Cette séparation est classiquement effectuée à l'aide de décanteurs gravitaires, appelés également ballons séparateurs. Ils sont mis en œuvre pour séparer le brut de pétrole et la phase aqueuse et se présentent généralement sous la forme de réservoirs de volume interne important au sein desquels circule continûment le fluide multiphasique dont on souhaite séparer les phases. Le temps de séjour du fluide multiphasique (émulsion) au sein du décanteur doit être suffisamment long pour permettre aux phases de différentes densités de se séparer par gravité. Ce temps constitue donc un paramètre important dans le fonctionnement du décanteur. Un exemple classique d'un décanteur est celui formé d'une chambre principale divisée en deux sous-chambres séparées par une paroi ouverte en partie supérieure pour laisser communiquer les deux sous-chambres. L'émulsion est introduite au sein de la première sous-chambre et seule l'une des deux phases est censée se diriger vers la seconde sous-chambre. Les deux phases ainsi séparées sont ensuite continûment évacuées hors du décanteur par des sorties correspondantes ménagées respectivement dans la première et dans la deuxième sous-chambre. Au sens de l'invention, une phase séparée peut tout de même contenir des quantités résiduelles d'une autre phase dudit fluide. Bien évidemment d'autres types de décanteurs existent et l'invention n'est pas limitée à un type de décanteur particulier.

Les décanteurs pétroliers fonctionnent généralement sous haute pression et haute température. Classiquement, les décanteurs utilisés sont prévus pour imposer un temps de séjour d'environ une dizaine de minute en leur sein, à un débit par exemple de l'ordre de 750 m³/h, de sorte que le volume de la chambre principale du décanteur est généralement de l'ordre de 120 m³.

Par ailleurs, les propriétés physico-chimiques de l'émulsion et le débit à traiter conditionnent la conception du décanteur pétrolier en particulier en ce qui concerne le volume de la première sous-chambre. Les décanteurs pétroliers sont ainsi généralement conçus pour un site de production donné après une analyse préliminaire des premières quantités d'émulsion extraites du puits pétrolier, le plus souvent à l'issue de la phase d'exploration du gisement pétrolier. Les décanteurs sont ainsi conçus durant une phase de conception afin de séparer un certain type d'émulsion dont les proportions de phases organique et aqueuse sont connues.

Or, l'émulsion pétrolière entrant au sein du décanteur n'a pas des propriétés physico-chimiques constantes au cours du temps et ces propriétés peuvent même fortement varier. En particulier, le brut recueilli peut être de composition différente en fonction des diverses poches exploitées dans le gisement ou en fonction des différents additifs chimiques qu'il a été nécessaire d'injecter dans le puits pour permettre une meilleure récupération de ce pétrole. Par ailleurs, les gisements produisent généralement une émulsion majoritairement composée d'huile en début de production et majoritairement constituée d'eau en fin de vie.

Ainsi, le décanteur ne peut généralement pas fonctionner de manière optimale à un débit constant dans un site de production pétrolière, car l'émulsion entrant au sein du décanteur peut elle-même varier au cours de l'exploitation. Il est donc nécessaire de connaître l'évolution des caractéristiques de séparation de l'émulsion entrante au cours du temps pour pouvoir piloter le fonctionnement du décanteur. En connaissant parfaitement les propriétés de l'émulsion entrante, on peut dès lors soit ajuster le débit du flux d'émulsion entrant, afin de modifier le temps de séjour, soit introduire au sein ou en amont (selon le sens d'écoulement du fluide) du décanteur des additifs chimiques (de type démulsifiant) censés accélérer la séparation des différentes phases. Ces deux actions, éventuellement complémentaires, doivent permettre d'atteindre les objectifs visés en ce qui concerne la qualité des fluides en sortie du séparateur. Piloter le décanteur en jouant sur ces deux paramètres s'avère particulièrement coûteux (diminuer le débit signifie diminuer la production et la vente de pétrole tandis que l'ajout d'additifs chimiques implique un coût non négligeable et une mise au point délicate) et il est donc primordial que les mesures indiquant l'effet de la variation de l'un ou l'autre de ces paramètres sur la qualité des fluides en sortie du séparateur soient fiables.

Cependant, le pilotage du décanteur s'avère particulièrement complexe à mettre en œuvre car il n'existe pas à ce jour de méthodes fiables permettant une très bonne caractérisation de l'émulsion.

Une des méthodes utilisée consiste à effectuer différentes analyses physico-chimiques de l'émulsion entrante. Ces analyses sont effectuées sur un échantillon prélevé ponctuellement en amont du décanteur. L'échantillon prélevé est ensuite généralement envoyé dans un laboratoire au sein duquel on effectue notamment des mesures de densité, de viscosité ou de teneurs d'eau dans l'huile ou d'huile dans l'eau. Ces analyses de l'émulsion entrante sont généralement complétées par des mesures effectuées sur les phases séparées à la sortie du décanteur. A cette fin, on prélève un échantillon au niveau des différentes sorties du décanteur et on envoie lesdits échantillons en laboratoire. En fonction de l'analyse de l'ensemble des résultats concernant l'émulsion entrante et les phases séparées sortantes, des ajustements en matière de conditions opératoires (débit ou ajouts d'additifs) sont proposés pour améliorer le fonctionnement du décanteur.

Mais une telle méthode ne s'avère pas être optimale pour les raisons suivantes.

Tout d'abord, il s'écoule nécessairement un laps de temps non négligeable entre le prélèvement et le résultat de la mesure, laps de temps pendant lequel l'émulsion traitée par le décanteur est susceptible de varier ou pendant lequel les propriétés physico-chimiques de l'échantillon prélevé peuvent varier, rendant ainsi certaines des mesures effectuées caduques, ou du moins difficilement exploitables. Cela constitue bien entendu un défaut important d'une telle méthode d'ajustement du fait du défaut de réactivité qui en découle. En particulier, pendant le transport, pressions et températures varient et la séparation des phases s'effectue de manière particulière et différente du comportement de l'émulsion au sein de l'installation industrielle.

De plus, ces analyses permettent simplement de mesurer certaines propriétés de l'émulsion et ne donnent pas une mesure du comportement de l'émulsion lorsque cette dernière circule au sein de l'installation. Par ailleurs, ces mesures ne peuvent en aucun cas anticiper un dysfonctionnement futur du décanteur et ne sont en réalité que des indicateurs grossiers pour piloter le fonctionnement de l'installation.

Par ailleurs, les mesures ainsi effectuées ne permettent pas de caractériser indépendamment les différents phénomènes physiques intervenant lors du processus de séparation des phases sous l'effet de la gravité. En effet, la séparation est en premier lieu conditionnée par la décantation qui est la propension des gouttelettes d'une phase dans une autre à se séparer sous l'effet de la gravité ; en second lieu la séparation dépend de la coalescence qui le phénomène par lequel deux gouttes d'une même phase se réunissent en une seule. Les analyses effectuées sur des échantillons prélevés ne permettent pas de discriminer ces deux phénomènes (décantation ou coalescence) ce qui constituerait pourtant une information précieuse pour caractériser le fluide multiphasique et ainsi correctement piloter l'installation au sein de laquelle il circule.

De plus, cette méthode reposant sur la réalisation de mesures sur échantillons ne permet pas non plus de s'assurer aisément et de façon fiable de la qualité des solutions mises en œuvre (ajout d'additifs ou variation du débit).

Il convient également de noter que des problèmes similaires peuvent être rencontrés dans d'autres secteurs d'activité que le domaine pétrolier, comme par exemple dans le domaine du traitement des eaux usées, notamment dans les stations d'épuration reposant sur la mise en œuvre de décanteurs. Il s'avère en effet extrêmement important de caractériser le fluide entrant au sein d'une installation de traitement afin de pouvoir proposer une solution optimale pour ce traitement, quel qu'il soit.

En définitive, il n'existe pas à ce jour de moyens permettant de caractériser correctement le comportement d'un fluide multiphasique afin par exemple de piloter efficacement le fonctionnement d'une installation de traitement d'un fluide multiphasique.

Le document US-2005/0016292 décrit un système de mesure de débit multiphasique mettant en œuvre un séparateur de phases à vortex.

### EXPOSE DE L'INVENTION

Les objets assignés à la présente invention visent en conséquence à remédier aux différents inconvénients énumérés précédemment et à proposer une nouvelle installation de traitement d'un fluide multiphasique dont le pilotage du fonctionnement est optimisé et permettant d'apporter rapidement et sans perturbation de l'exploitation une information utile pour le pilotage du fonctionnement de l'installation.

Un autre objet de l'invention vise à proposer une nouvelle installation de traitement d'un fluide multiphasique dont le fonctionnement est particulièrement fiable, et qui permet d'obtenir de meilleures séparation et cadences de production.

Un autre objet de l'invention vise à proposer une nouvelle installation de traitement d'un fluide multiphasique aisément adaptable à toute unité de production existante.

Un autre objet de l'invention vise à proposer une nouvelle installation de traitement d'un fluide multiphasique de conception particulièrement simple et robuste.

Un autre objet de l'invention vise à proposer un nouveau procédé de caractérisation d'un fluide multiphasique permettant d'optimiser le pilotage du fonctionnement d'une installation traitant un tel fluide.

Un autre objet de l'invention vise à proposer un nouveau procédé de caractérisation d'un fluide multiphasique permettant de contrôler la séparation d'un tel fluide au sein d'une installation de traitement.

Les objets assignés à l'invention sont atteints à l'aide d'une installation selon la revendication 1.

Les objets assignés à l'invention sont également atteints à l'aide d'un procédé selon la revendication 13.

### DESCRIPTIF SOMMAIRE DES DESSINS

D'autres objets et avantages de l'invention apparaîtront mieux à la lecture de la description ci-après, ainsi qu'à l'aide des dessins annexés, donnés à titre purement illustratif et non limitatif, dans lesquels :
- La figure 1 illustre, selon une vue schématique en coupe, un exemple d'une installation de traitement d'un fluide multiphasique conforme à l'invention, ladite installation étant en cours de fonctionnement et incluant un décanteur principal et un décanteur d'analyse.
- La figure 2 représente, selon une vue schématique en coupe, un décanteur d'analyse de l'installation de la figure 1, ledit décanteur d'analyse étant en cours de fonctionnement.
- La figure 3 illustre, selon une vue schématique en coupe, un second exemple de décanteur d'analyse faisant partie d'une installation selon l'invention conçue pour séparer trois phases liquides d'un fluide multiphasique, ledit décanteur d'analyse étant en cours de fonctionnement.

### MEILLEURE MANIERE DE REALISER L'INVENTION

La présente invention concerne une installation de traitement 1 d'un fluide multiphasique comprenant au moins deux phases de densités respectives différentes, et en particulier au moins deux phases liquides de densités respectives différentes, appelées respectivement dans ce qui suit « *première phase* » et « *deuxième phase* ». Généralement, une des phases est dispersée dans l'autre. Au sens de l'invention, un liquide constitue une phase lorsque les différents éléments le composant ne peuvent pas être séparés par gravité. Ainsi, un mélange de liquides miscibles constitue une seule et même phase.

A titre d'exemple, l'installation de traitement 1 selon l'invention peut constituer un élément d'une unité de production pétrolière. Dans cette situation, l'objectif de l'installation 1 conforme à l'invention est de récupérer les phases valorisables (pétrole) du fluide multiphasique et de rejeter vers l'extérieur, ou vers d'autres installations, les autres phases (eau, etc.). A titre alternatif, l'installation de séparation 1 peut par exemple constituer un élément d'une station d'épuration. Dans ce cas, le fluide multiphasique comprend des eaux altérées par des activités humaines à la suite d'un usage domestique, industriel ou agricole. La première phase est alors constituée par de l'eau que l'on souhaite récupérer tandis que les déchets et/ou les polluants constituent la seconde phase. Pour des questions de concision et de simplicité de description, la description qui suit sera focalisée sur l'exemple préférentiel d'une installation 1 formant un élément d'un site de production pétrolière. Bien évidemment, l'invention n'est pas limitée à cette application préférentielle, et toute autre situation dans laquelle il est nécessaire de traiter un fluide multiphasique comportant des phases liquides de densités différentes rentre dans le cadre de l'invention.

Afin de récupérer la ou les phase(s) valorisable(s), l'installation de traitement 1 d'un fluide multiphasique comprend un décanteur principal 2 destiné à séparer au moins lesdites phases liquides. Le décanteur principal 2 est donc un décanteur de production destiné à produire de façon industrielle un liquide valorisable. Dans le cas de l'application préférentielle de l'invention, savoir la production pétrolière, le fluide multiphasique pénétrant au sein du décanteur principal 2 est une émulsion comportant une phase organique liquide (contenant le pétrole qui est la phase valorisable), formant ladite première phase, une phase aqueuse liquide, formant ladite seconde phase et une phase gazeuse. La fonction première du décanteur principal 2 est en l'espèce de séparer les deux phases liquides, la phase gazeuse étant quant à elle extraite du décanteur principal 2, par l'intermédiaire d'un évent 22C ménagé au sommet du décanteur principal 2 et communiquant avec une installation de traitement de gaz pour valoriser ou brûler ces derniers. L'installation de traitement 1 constitue donc une installation de séparation permettant de produire un liquide valorisable par séparation (en l'espèce gravitaire) d'au moins deux phases liquides d'un fluide primaire multiphasique.

Comme illustré aux figures, l'installation 1 selon l'invention comprend un circuit 4 au sein duquel est destiné à circuler le fluide multiphasique, ledit circuit 4 comprenant en l'espèce ledit décanteur principal 2. Le circuit 4 est constitué d'un ensemble de canalisations et de dispositifs divers, au sein desquels circule le fluide. Il délimite donc le chemin suivi par le fluide multiphasique pour être transformé. Dans l'exemple préférentiel d'application de l'invention (production pétrolière), le circuit 4 s'étend au moins en partie entre le gisement de pétrole et le lieu de livraison du brut de pétrole. Il comprend donc les lieux par lesquels ledit fluide multiphasique s'écoule, et dont fait notamment partie le décanteur principal 2 comme exposé précédemment. De manière préférentielle, le circuit 4 comprend au moins un conduit d'acheminement 5 de fluide multiphasique permettant notamment l'alimentation en fluide multiphasique du décanteur principal 2 pour convoyer, de préférence de façon continue, un flux de fluide multiphasique vers et dans le décanteur principal 2. Le conduit d'acheminement 5 peut également être relié à d'autres éléments de l'installation 1 ou du site industriel auquel appartient l'installation 1. Il peut notamment être formé de tuyaux et de jonctions au sein desquels circule le fluide multiphasique.

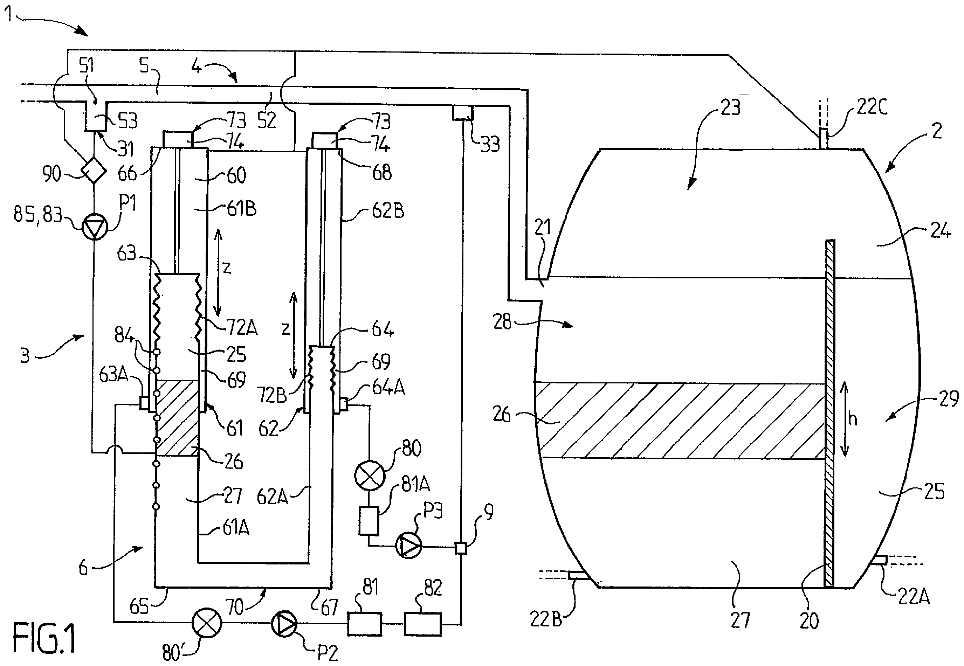

Un exemple d'une installation de séparation 1 selon l'invention est illustré de manière générale et schématique à la figure 1. L'installation de séparation 1 comprend avantageusement un décanteur principal 2 destiné à séparer au moins lesdites phases liquides dudit fluide multiphasique comme décrit ci-avant. Ce décanteur principal 2 comprend au moins une entrée 21 par laquelle le fluide multiphasique est destiné à pénétrer dans le décanteur principal 2, pour alimenter ce dernier. L'entrée 21 forme ainsi l'interface de jonction entre le volume intérieur du décanteur principal 2 et l'extérieur (*i.e.* le reste du circuit 4). Préférentiellement, le conduit d'acheminement 5 est relié à l'entrée 21 du décanteur principal 2, l'alimentation en fluide se faisant préférentiellement dans la couche liquide. Le décanteur principal 2 comprend également au moins une sortie 22A, 22B, 22C par laquelle ledit fluide est destiné à être évacué hors du décanteur principal 2, en l'espèce sous forme de phases séparées. Ainsi, lorsque le décanteur principal 2 est en fonctionnement, le fluide multiphasique circule continûment en son sein. Avantageusement le décanteur principal 2 est un décanteur industriel, c'est-à-dire qu'il constitue un décanteur de production adapté, en terme de capacité et d'aménagements intérieurs notamment, à traiter des quantités industrielles de fluide multiphasique (émulsion pétrolière ou eaux usées par exemple).

De préférence, le décanteur principal 2 est un décanteur gravitaire, de type ballon séparateur. Il se présente sous la forme d'un réservoir (dont l'enveloppe est généralement métallique) au sein duquel circule continûment le fluide multiphasique dont on souhaite séparer les phases. La capacité du réservoir et la vitesse de déplacement du fluide multiphasique sont telles que le temps de séjour du fluide multiphasique (émulsion) au sein du décanteur 2 est suffisamment long pour permettre aux phases liquides de différentes densités de se séparer par gravité. Le décanteur principal 2 comprend avantageusement une chambre de séparation 23 au sein de laquelle la séparation des différentes phases du fluide multiphasique a lieu. La gravité est le phénomène physique principal qui permet cette séparation de la façon suivante : le fluide multiphasique pénètre au sein du décanteur et reste à l'intérieur durant un certain laps de temps, appelé temps de séjour ; ce temps de séjour est suffisamment long pour permettre aux différentes phases liquides de se séparer en s'étageant verticalement en fonction de leurs densités respectives. Les phases liquides séparées sont ensuite évacuées séparément hors du décanteur principal 2, par des sorties respectives 22A, 22B. Au sens de l'invention, une phase séparée peut tout de même contenir des quantités résiduelles d'une autre phase dudit fluide. Avantageusement, le décanteur principal 2 comprend également une paroi interne 20, avantageusement ouverte en partie supérieure, permettant de délimiter au sein de la chambre de séparation 23 deux sous-chambres 28, 29 conçues pour permettre la sortie de chacune des phases à séparer, étant entendu que le décanteur principal 2 comprend en outre à cet effet des sorties 22A, 22B, 22C destinées à permettre l'évacuation des phases séparées. La figure 1 illustre un exemple de décanteur principal 2 comprenant trois sorties : la première sortie 22B est destinée à évacuer la phase liquide la plus dense (généralement la phase aqueuse), la deuxième sortie 22A est destinée à extraire la phase organique liquide (comprenant le pétrole) et la troisième sortie 22C est destinée à évacuer la phase gazeuse. Bien évidemment, d'autres types de décanteurs existent et l'invention n'est pas limitée à un type de décanteur particulier, qu'il comprenne ou non une paroi interne 20.

La séparation au sein d'un unique décanteur principal 2 peut s'avérer être plus ou moins imparfaite. Il est donc envisageable, sans pour autant sortir du cadre de l'invention, de brancher plusieurs décanteurs 2 en série, ou de manière plus générale en cascade, pour obtenir *in fine* un meilleur degré de séparation.

De manière préférentielle, le décanteur principal 2 est de forme cylindrique, ce qui lui permet notamment de mieux supporter la forte pression susceptible de régner en son sein (l'extraction pétrolière s'effectue généralement à une pression comprise entre 10 et 40 bars). L'axe de révolution du cylindre X-X' peut être vertical ou horizontal, en fonction du type de décanteur principal 2 considéré. Avantageusement, le décanteur principal 2 est un cylindre de base circulaire, dont le diamètre du cercle générateur est compris par exemple entre 1 et 4 m, qui s'étend par exemple selon une longueur comprise entre 5 et 30 m. Le fonctionnement et les différentes variantes de décanteurs de production sont parfaitement connus en tant que tels et ne nécessitent donc pas d'être décrits plus en détails ici. L'installation 1 selon l'invention n'est ainsi absolument pas limitée à un type spécifique de décanteur principal 2. Par exemple, le décanteur principal 2 selon l'invention peut être fixe et être uniquement destiné à être positionné à demeure au sein d'une unité industrielle. De façon alternative, le décanteur principal 2 peut également être mobile pour pouvoir être transporté par exemple sur le lieu d'une marée noire et séparer les phases de l'émulsion formant cette marée noire.

Après avoir pénétré au sein du décanteur principal 2, l'émulsion reste durant un certain temps de séjour en son sein. Ce temps de séjour va permettre aux gouttes de la phase légère (généralement la phase organique) de remonter à la surface et aux gouttes de la phase plus dense (généralement la phase aqueuse) de descendre vers le fond de la chambre. Plus les gouttes seront fines, plus ce processus sera lent. En outre, plus la coalescence des gouttes d'une même phase sera rapide, plus le processus de séparation sera court. Au sein du décanteur principal 2, le fluide multiphasique est dissocié en différents secteurs dont les compositions sont clairement différentes. Ces secteurs sont étagés verticalement au sein du décanteur principal 2. Le secteur supérieur 24 comprend la phase gazeuse tandis que le secteur inférieur comprend les phases liquides. La phase gazeuse est évacuée hors du décanteur principal 2 *via* un système d'évacuation dédié 22C, comme par exemple un évent. Le secteur inférieur est quant à lui subdivisé en trois zones superposées. La zone supérieure 25 comporte la phase la plus légère qui correspond par exemple à la phase organique liquide (huile). La zone inférieure 27 comporte la phase la plus dense qui correspond par exemple à la phase aqueuse. Entre ces deux zones se situe généralement une zone intermédiaire 26, formant une couche entre lesdites phases aqueuse et organique, comprenant un mélange de ces dernières. Cette couche est notamment appelée « *couche de coalescence* ». L'étagement de ces différents secteur 24 et zones supérieure 28, intermédiaire 26, inférieure 27 est notamment illustré sur la vue en coupe du décanteur principal 2 de la figure 1.

L'invention découle notamment du constat que les propriétés de la zone intermédiaire 26 dépendent des propriétés physico-chimiques de l'émulsion entrante. Dans certains cas, l'aspect de la zone intermédiaire est assimilable à celui d'une mousse. Or, il a été constaté que le drainage de cette mousse pouvait se révéler être parfois le processus le plus long de la séparation. Cela a mis en évidence l'intérêt qu'il y a de se concentrer notamment sur l'étude du comportement de cette zone intermédiaire 26 afin de caractériser la séparation des phases du fluide multiphasique. Avantageusement, l'étude de cette zone 26 permet également d'améliorer l'efficacité de la séparation au sein du décanteur principal 2. La hauteur h (épaisseur) de cette zone 26 va jouer un rôle significatif sur les performances du décanteur principal 2. Connaître les propriétés de cette zone 26 se révèle dès lors important pour analyser le fonctionnement du décanteur principal 2.

Mais une étude directe est complexe à réaliser car les mesures internes au décanteur principal 2 sont difficiles à mettre en œuvre, surtout si l'on souhaite éviter de perturber la production, ce qui est primordial. Il est donc important de s'intéresser aux propriétés de séparation du fluide multiphasique pour piloter correctement le fonctionnement du décanteur principal 2. De manière préférentielle, l'invention a pour objectif de caractériser la séparabilité gravitaire du fluide multiphasique circulant au sein de l'installation 1 Au sens de l'invention, la séparabilité gravitaire d'un fluide multiphasique caractérise la vitesse à laquelle les différentes phases constitutives du mélange se séparent sous l'effet de la gravité L'invention consiste donc à chercher à caractériser la séparabilité d'une phase de l'émulsion dans une autre, c'est-à-dire, le temps nécessaire pour une phase présente dans l'émulsion pour se séparer de l'émulsion. Pour cela, il est non seulement nécessaire de s'intéresser à cette couche de coalescence, la zone 26, mais aussi de connaître les propriétés de décantabilité des gouttes d'une phase dans une autre, c'est-à-dire la propension des gouttes d'une phase présentes dans une autre phase à se séparer sous l'effet de la gravité. La caractérisation de ces deux phénomènes permet de caractériser la séparabilité gravitaire de l'émulsion.

L'installation de séparation 1 selon l'invention comprend également un outil de caractérisation 3 du fluide multiphasique. Cet outil de caractérisation 3 a pour objectif de permettre l'accès à des paramètres déterminant le comportement du fluide multiphasique lors de la séparation de ses phases sous l'effet de la gravité. Avantageusement, ces paramètres sont susceptibles de refléter et/ou d'influer sur le fonctionnement et les possibles dysfonctionnements du décanteur principal 2. L'outil de caractérisation 3 est alors conçu pour fournir une aide à la prise de décision pour le pilotage du fonctionnement du décanteur principal 2. Plus généralement, il permet de caractériser les propriétés d'une émulsion. L'outil de caractérisation 3 comprend au moins une ouverture d'entrée 31, et une ouverture de sortie 33, toutes deux connectées audit circuit 4. Plus précisément, l'ouverture d'entrée 31 et l'ouverture de sortie 33 sont respectivement connectées au circuit 4 en amont de ladite entrée 21 du décanteur principal 2 et en amont de ladite sortie 22A, 22B, 22C du décanteur principal 2, relativement au sens d'écoulement dudit fluide multiphasique au sein du circuit 4. Au sens de l'invention, l'outil de caractérisation 3 ne fait pas lui-même partie du circuit 4 et est simplement connecté à ce dernier. L'outil de caractérisation 3 est donc branché, de préférence de manière permanente et continue, en dérivation du circuit 4, de sorte qu'une fraction, de préférence minoritaire, du flux de fluide multiphasique circulant dans le circuit 4 est déporté dans l'outil de caractérisation 3 avant d'être ré-introduite dans le circuit 4. Cette dérivation est en l'espèce réalisée grâce au fait que les ouvertures d'entrée 31 et de sortie 33 sont toutes deux connectées au circuit 4, l'ouverture de sortie 33 pouvant être positionnée en amont ou en aval, mais préférentiellement en aval, de ladite ouverture d'entrée 31, dans le sens d'écoulement du fluide multiphasique au sein du circuit 4. Cette dérivation partielle du flux de fluide circulant au sein du circuit 4 vers l'outil de caractérisation 3 garantit le fait que l'analyse du fluide multiphasique au sein de l'outil de caractérisation 3 donnera des résultats représentatifs en temps réel du fluide traité au sein de l'installation 1. Cette dérivation permet également d'effectuer des mesures sur l'émulsion entrant au sein du circuit 4 sans perturber l'écoulement continu du fluide multiphasique au sein de l'installation 1. Déporter une fraction minoritaire et de préférence insignifiante (en termes de quantité) du flux de l'émulsion entrante pour l'analyser permet ainsi d'effectuer des mesures de manière rapide, continue et efficace sur le fluide multiphasique. Cela constitue un des apports de l'invention. Par ailleurs, l'outil de caractérisation 3 est conçu de telle sorte que le fluide ayant circulé en son sein soit rejeté dans le circuit 4 dans le, ou à l'amont du, décanteur principal 2, sans perturber le fonctionnement de l'installation.

Comme exposé précédemment, l'ouverture d'entrée 31 et l'ouverture de sortie 33 sont connectées respectivement en amont de ladite entrée 21 et en amont de ladite sortie 22A, 22B, 22C, relativement au sens d'écoulement dudit fluide multiphasique au sein du circuit 4, de façon à dériver, de préférence de façon permanente, continue, une partie du fluide multiphasique circulant dans le circuit 4 vers et dans l'outil d'analyse 3. Ainsi, le fluide multiphasique pénétrant au sein de l'outil de caractérisation 3 est donc sensiblement similaire au fluide multiphasique pénétrant au sein du décanteur principal 2. Les résultats obtenus par l'étude du fluide multiphasique circulant au sein de l'outil de caractérisation 3 sont donc aisément transposables pour l'étude du fonctionnement du décanteur principal 2.

Avantageusement, le conduit d'acheminement 5 est pourvu d'un embranchement 51 divisant ledit conduit 5 en au moins une première branche 52 et une deuxième branche 53. Lesdites première et deuxième branches 52, 53 sont avantageusement constituées chacun d'un ou plusieurs tuyau(x) permettant la circulation du fluide multiphasique en provenance du site d'extraction vers respectivement le décanteur principal 2 et l'outil d'analyse 3. La première branche 52 est préférentiellement connectée à ladite entrée 21 tandis que ladite deuxième branche 53 est connectée à ladite ouverture d'entrée 31 pour permettre l'alimentation concomitante en fluide multiphasique du décanteur principal 2 et de l'outil d'analyse 3. Les première et deuxième branches 52, 53 peuvent également être subdivisées en différentes branches ou connectées à d'autres parties du circuit 4 sans que l'on sorte du cadre de l'invention. En d'autres termes, l'installation 1 est conçue pour dériver une fraction du fluide multiphasique circulant dans le circuit 4 vers et dans l'outil de caractérisation 3, de façon que le décanteur principal 2 et l'outil de caractérisation 3 soient alimentés concomitamment en fluide multiphasique.

Come exposé précédemment, l'ouverture de sortie 33 est quant à elle connectée en amont de ladite sortie 22A, 22B, 22C dans le sens d'écoulement du fluide multiphasique au sein du circuit 4 de façon à réintroduire le fluide multiphasique qui a traversé l'outil de caractérisation 3 dans le circuit 4. Cela permet d'éviter la perte de phases aqueuse et organique issues du passage du fluide multiphasique à travers le décanteur d'analyse 6. Avantageusement, l'ouverture de sortie 33 est connectée audit conduit 5 et/ou audit décanteur principal 2 de façon à réinjecter le fluide dérivé du circuit 4 dans le circuit 4, soit directement dans le décanteur principal 2, soit en amont du décanteur principal 2, pour qu'au moins une fraction du fluide ainsi réinjecté soit traité par le décanteur principal 2. L'injection peut s'effectuer en tout lieu en amont de la sortie du décanteur principal 2, de façon que les différentes phases du fluide ayant circulé au sein de l'outil de caractérisation 3 puissent être par la suite séparées par le décanteur principal 2.

Avantageusement, la phase gazeuse présente au sein de l'émulsion est au moins partiellement pré-séparée dans l'outil de caractérisation 3, avant que le fluide multiphasique ne pénètre dans le décanteur d'analyse 6. Cette phase gazeuse pré-séparée est ensuite réinjectée dans le circuit 4, préférentiellement vers la sortie 22C, destiné à évacuer le gaz du décanteur principal 2. Préférentiellement, cette opération est réalisée à l'aide d'un pré-séparateur 90 situé au niveau ou en aval de l'ouverture d'entrée 31. De façon alternative, cette phase gazeuse pré-séparée peut également être évacuée par une sortie spécifique qui fait partie du circuit 4 au sens de l'invention. Avantageusement, l'outil de séparation 3 comprend également une ou plusieurs sorties reliées à ladite sortie spécifique et qui permet d'évacuer la phase gazeuse.

L'outil de caractérisation 3 selon l'invention comprend par ailleurs d'une part un décanteur d'analyse 6, disposé entre lesdites ouvertures d'entrée 31 et de sortie 33, et apte à séparer lesdites phases liquides pour obtenir des phases dites séparées, et d'autre part un moyen de réunion 9 desdites phases séparées. L'installation 1 est conçue pour que ladite fraction du fluide circulant au sein de l'outil 3 pénètre par ladite ouverture d'entrée 31 pour passer par ledit décanteur d'analyse 6 afin que lesdites phases soient séparées puis évacuées par ladite ouverture de sortie 33 dans ledit circuit 4, ledit moyen de réunion 9 étant conçu pour réunir lesdites phases séparées à l'aval du décanteur d'analyse 6 et en amont de ladite sortie 22A, 22B, 22C du décanteur principal 2 par rapport au sens d'écoulement du fluide. Le moyen de réunion 9 permet de remélanger les phases au sein du fluide multiphasique. Ainsi le décanteur d'analyse 6, bien que séparant les phases, n'a pas d'impact sur la composition du fluide multiphasique circulant au sein du circuit 4. Ce moyen de réunion 9 est avantageusement formé par une connexion, située en amont de l'ouverture de sortie 33, des conduits acheminant les différentes phases issues du décanteur d'analyse 6. Dans cette situation, les phases sont injectées dans le circuit 4 en étant déjà réunies. De manière alternative, les phases peuvent être rejetées en des lieux différents du circuit 4. Dans ce cas-là, le moyen de réunion 9 est formé par le conduit du circuit 4 où les phases se réunissent. Les phases séparées se mélangent au sein du fluide multiphasique et ne sont pas injectées dans le circuit 4 en étant réunies mais en étant séparées. Ainsi, l'outil de caractérisation 3 n'a pas d'impact direct sur le fonctionnement de l'installation 1, il sert uniquement à fournir des informations sur le fluide multiphasique circulant en son sein. Ces informations peuvent ensuite être utiles pour améliorer le fonctionnement de l'installation 1

Avantageusement, l'outil de caractérisation 3 comprend un moyen de limitation du débit 83 en fluide multiphasique pénétrant au sein dudit outil 3 de façon que le débit de fluide multiphasique entrant au sein dudit outil de caractérisation 3 soit inférieur au débit de fluide multiphasique circulant dans ledit circuit 4 au niveau de ladite ouverture d'entrée 31. En pratique, l'introduction du fluide multiphasique dans le décanteur d'analyse 6 est de préférence effectuée à l'aide d'une pompe P1, de préférence volumétrique, de telle sorte que le débit d'alimentation de l'outil d'analyse puisse être aisément réglé par variation de la vitesse de rotation de la pompe 83. Ainsi, le débit du flux de fluide multiphasique circulant au sein de l'outil de caractérisation 3 est de préférence beaucoup plus faible que celui circulant au sein du circuit 4 au niveau de l'ouverture d'entrée 31, et de sorte qu'avantageusement les temps de séjours dans l'outil de caractérisation 3 et dans le décanteur principal 2 soient comparables. Le fait de dériver une partie restreinte du flux total du fluide multiphasique entrant permet de réaliser facilement les mesures nécessaires à l'analyse de ce fluide sans risquer de compromettre les transformations opérées au sein de l'installation de traitement 1.

Préférentiellement, ce moyen de limitation 83 permet de réduire le débit d'un facteur 10, ou de manière plus préférentielle, d'un facteur 100, voire 100000, par rapport au débit circulant au sein du circuit 4, au niveau de l'ouverture d'entrée 31.

De manière préférentielle, l'évacuation du fluide multiphasique hors du décanteur d'analyse 6 est effectuée à l'aide de pompes P3, P4 dont le débit est ajusté de manière à stabiliser les volumes des phases séparées contenus dans le décanteur d'analyse 6, et notamment de les réguler par ajustement des vitesses de rotation desdites pompes P3, P4.

Avantageusement, le décanteur d'analyse 6 fonctionne en parallèle du décanteur principal 2. Ainsi, le fluide circule non seulement au sein du décanteur principal 2 mais également au sein de l'outil de caractérisation 3, en pénétrant par ladite ouverture d'entrée 31, en passant par ledit décanteur d'analyse 6 et en étant évacué par ladite ouverture de sortie 33 vers le circuit 4. Ainsi, l'outil de caractérisation 3 évalue les propriétés du fluide multiphasique lorsque ce dernier s'écoule en son sein.

Préférentiellement, le fluide circule donc avantageusement continûment à la fois au sein du décanteur principal 2 et du décanteur d'analyse 6, sous la forme de deux flux parallèles, savoir un flux de production (majoritaire) et un flux d'analyse (minoritaire). Une des fonctions du décanteur d'analyse 6 est de pouvoir séparer les phases du fluide multiphasique en parallèle du décanteur principal 2 en vue notamment de pouvoir aisément réaliser des mesures physico-chimiques sur ces phases ainsi séparées. Cela permet d'accéder aux diverses propriétés de ces phases en continu et en temps réel.

Le décanteur d'analyse 6 permet avantageusement de simuler au moins en partie et en temps réel le fonctionnement du décanteur principal 2. Le décanteur d'analyse 6 permet ainsi d'accéder à différents paramètres relatifs à un flux de fluide similaire à celui pénétrant au sein du décanteur principal 2 puisque dérivé du flux de fluide alimentant le décanteur principal 2, et ce sans perturber le fonctionnement du décanteur principal 2. Le décanteur d'analyse 6 est de préférence conçu pour offrir des conditions de décantation analogues ou proches de celles du décanteur principal 2, à un éventuel effet d'échelle près, sans que cela implique pour autant un mimétisme absolu, l'essentiel étant que les conditions de décantation soient suffisamment proches pour que la séparation se déroulant au sein du décanteur d'analyse 6 fournisse une information pertinente pour caractériser l'émulsion ainsi que pour analyser le fonctionnement du décanteur principal 2.

Le décanteur d'analyse 6 comprend avantageusement une chambre de séparation 60 au sein de laquelle le fluide multiphasique est destiné à circuler afin que ses phases soient séparées sous l'effet de la gravité. Avantageusement, le volume de la chambre de séparation 23 du décanteur principal 2 est au moins dix fois plus grand que le volume de la chambre de séparation 60 du décanteur d'analyse 6, préférentiellement au moins cent fois plus grand.

Avantageusement, le décanteur d'analyse 6 fonctionne sur le principe du puits florentin et constitue de préférence un tel puits florentin.

De manière préférentielle, ledit décanteur d'analyse 6 comprend au moins d'une part un canal de séparation 61 pourvu d'un premier orifice de sortie 63 et s'étendant verticalement entre une première base 65 et un premier sommet 66 et d'autre part un canal d'écoulement 62 pourvu d'un second orifice de sortie 64 et s'étendant verticalement entre une seconde base 67 et un second sommet 68. Le premier canal de séparation 61 est connecté à l'ouverture d'entrée 31 pour alimenter le décanteur d'analyse 6 en fluide. La séparation en différentes phases du fluide multiphasique s'effectue au sein du canal de séparation 61 tandis que le canal d'écoulement 62 est destiné à l'évacuation de l'une desdites phases (de la phase dense). Les canaux de séparation 61 et d'écoulement 62 peuvent être de forme quelconque, par exemple cylindrique ou parallélépipédique, l'essentiel étant qu'ils présentent une certaine extension verticale (hauteur) pour permettre la décantation du fluide en leur sein.

Les orifices de sortie 63, 64 constituent les lieux par lesquels est évacué le fluide multiphasique hors du décanteur d'analyse 6. De manière préférentielle, l'évacuation est effectuée par déversement du fluide au niveau des orifices de sortie 63, 64. Ces derniers sont préférentiellement reliés à l'ouverture de sortie 33. Ils peuvent être formés par exemple d'un trou, avantageusement de dimension suffisamment large pour permettre un écoulement libre par déversement, ménagé au sein de chaque canal de séparation 61 et d'écoulement 62.

Préférentiellement, les canaux de séparation 61 et d'écoulement 62 sont formés de tubes télescopiques 61A, 61B 62A, 62B joints de manière étanche. Les tubes de diamètre supérieur 61B, 62B recouvrent au moins en partie les tubes de diamètre inférieur 61A, 62A. Un interstice 69 est ainsi formé par les parois des tubes de diamètre inférieur 61A, 62A et des tubes supérieurs 61 B, 62B au niveau de ce recouvrement. Les tubes de diamètre inférieur 61A, 62A forment la partie inférieure des canaux de séparation 61 et d'écoulement 62 et le fluide multiphasique circule en leur sein. Les orifices de sortie 63, 64 sont dès lors respectivement formés par les orifices terminaux des tubes de diamètre inférieur 61A, 62A et forment ainsi des déversoirs. Le fluide peut ainsi déborder des tubes de diamètre inférieur 61A, 62A par les orifices de sortie 63, 64 pour circuler dans l'interstice 69, entre les tubes de diamètre inférieur 61A, 62A et de diamètre supérieur 61 B, 62B, jusqu'à une connexion 63A, 64A reliés à ladite ouverture de sortie 33 par l'intermédiaires des pompes P2 et P3. Dans ce cas, l'altitude respective des orifices de sortie 63, 64 au sein de chaque canal de séparation 61 et d'écoulement 62 définit la hauteur maximale que peut atteindre le fluide au sein de chaque canal de séparation 61 et d'écoulement 62. Une telle solution présente des avantages lorsque les positions des orifices de sortie 63, 64 sont mobiles, solutions présentées ci-après.

Le décanteur d'analyse 6 comprend également une connexion de raccord 70 connectant de manière étanche lesdits premier et second canaux de séparation 61, 62 vers leurs bases 65, 67. Cette connexion 70 définit un volume commun aux deux canaux de séparation. Le fluide multiphasique peut ainsi circuler au sein de ce volume commun et notamment passer d'un canal de séparation à un autre. Préférentiellement seule la phase la plus dense circule au sein de ce raccord 70, la séparation se faisant au niveau du canal de séparation 61.

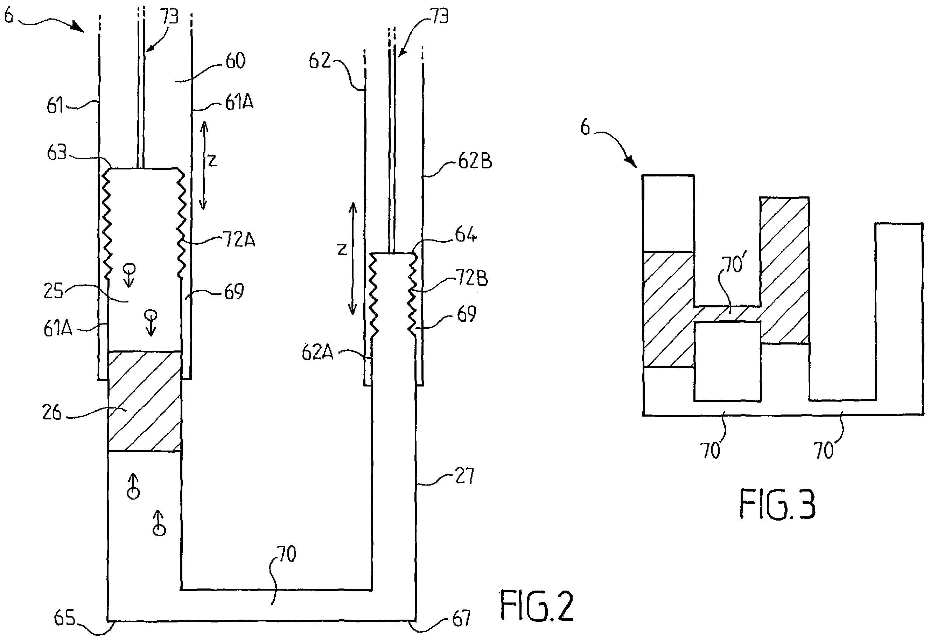

De manière préférentielle, le décanteur d'analyse 6 est formé de deux canaux de séparation 61 et d'écoulement 62 qui sont des colonnes reliées entre elles pour former un « *U* »*.* Les colonnes sont avantageusement des cylindres verticaux de même hauteur. Leur diamètre peut être différent. La figure 2 représente notamment le cas où le diamètre du canal de séparation 61 est supérieur au diamètre du canal d'écoulement 62. Les bras du « *U* » correspondent aux canaux de séparation 61 et d'écoulement 62 tandis que l'âme du « *U* » correspond à la connexion de raccord 70.

Préférentiellement, les canaux de séparation 61 et d'écoulement 62 sont d'une hauteur comparable à celle du décanteur principal 2. En effet, il peut être avantageux que le décanteur d'analyse 6 ait un comportement, au niveau de la séparation, similaire de celui du décanteur principal 2. Des dimensions verticales similaires permettent d'avoir des conditions de coalescence relativement proches, ou du moins représentatives, de celles présentes au sein du décanteur principal 2. De manière préférentielle, les pressions et les températures régnant au sein des deux décanteurs 2, 6 sont également sensiblement similaires. Pour réaliser cela, on isole notamment thermiquement l'outil de caractérisation 3 à l'aide de tout moyen connu d'isolation.

Les orifices de sortie 63, 64 sont avantageusement étagés verticalement de façon. séparer lesdites phases du fluide. Cet étagement permet en outre d'assurer la présence simultanée de couches 27, 28 et éventuellement 26 au sein du canal de séparation 61, selon le principe connu d'un puits florentin. Les altitudes des orifices de sortie 63, 64 sont donc différentes, l'altitude du premier orifice de sortie 63, à travers lequel est évacué la phase la moins dense (première phase) étant supérieure à celle du deuxième orifice de sortie 64, à travers lequel est évacué la phase plus dense (deuxième phase). L'altitude des différents orifices de sortie 63, 64 conditionne l'altitude de la zone intermédiaire 26 entre les deux phases liquides du fluide de densités différentes. En effet, les pressions régnant à l'intérieur des canaux 61 et d'écoulement 62 au niveau de chaque base 65, 67 des canaux de séparation 61 et d'écoulement 62 sont similaires car lesdites bases 65, 67 communiquent entre elles. Pour respecter cette continuité de pression, les différentes phases du fluide vont se superposer de manière particulière et ainsi positionner la zone intermédiaire 26 entre les différentes phases.

La position de la zone intermédiaire 26 ne dépend que de la densité des différentes phases et de la hauteur de fluide au sein des canaux de séparation 61 et d'écoulement 62. Il est donc nécessaire de correctement positionner les orifices de sortie 63, 64 pour s'assurer que cette zone 26 puisse bien être positionnée au sein du canal de séparation 61, celui dans lequel pénètre en premier le fluide multiphasique, et éviter ainsi tout dysfonctionnement majeur. En effet, si la zone intermédiaire 26 n'est pas positionnée dans le canal de séparation 61 (positionnée à une altitude trop basse), alors un mélange de phases est susceptible de pénétrer dans le canal d'écoulement 62 : on ne peut dès lors plus récupérer une phase aqueuse suffisamment pure et le décanteur d'analyse 6 ne fonctionne alors pas correctement. La possibilité de modifier le positionnement des orifices 63 et 64 permet notamment de caractériser la séparabilité du fluide multiphasique sans qu'intervienne un dysfonctionnement majeur au sein du décanteur d'analyse 6. Mais un tel dysfonctionnement peut également être simulé dans le but de caractériser le comportement du fluide multiphasique dans ce genre de situations qui peuvent se produire au sein du décanteur principal 2, ou afin de caractériser l'épaisseur de la couche de coalescence 26. On pourrait avoir un dysfonctionnement inverse si la zone intermédiaire était positionnée à une altitude trop haute.

Préférentiellement, le positionnement des orifices de sortie 63, 64 doit tenir compte des spécificités géométrique du décanteur principal 2, pour offrir au fluide multiphasique pénétrant au sein du décanteur d'analyse 6 des conditions de séparation similaires ou proches de celles rencontrées dans le décanteur principal 2. Ce positionnement doit également tenir compte de la densité respective de chacune des deux phases liquides séparées. Le décanteur principal 2 est dimensionné en fonction de l'émulsion entrante et le décanteur d'analyse 6 peut subir le même type de contrainte de dimensionnement, notamment pour les diamètres respectifs des canaux de séparation 61 et d'écoulement 62.

Toutefois, l'outil de caractérisation 3 peut également fonctionner selon une plage de fonctionnement beaucoup plus importante pour caractériser non pas le décanteur 2 mais le fluide multiphasique qui le traverse.

Avantageusement, l'outil de caractérisation 3 comprend un moyen de réglage 71 de la position verticale du premier et/ou du second orifice de sortie 63, 64. Grâce à cette fonctionnalité l'outil de caractérisation 3 est adaptable à n'importe quelle installation de traitement 1 ou n'importe quel décanteur principal 2 et à une large gamme d'émulsion en ce qui concerne la différence de densité. Il suffit pour cela de modifier la position des différents orifices de sortie 63, 64. Un seul et même outil de caractérisation 3 pourvu d'un tel moyen de réglage 71 peut être dès lors mis en place sur différentes installations de traitement 1.

De manière préférentielle, le canal de séparation 61 et/ou le canal d'écoulement 62 comprend (comprennent) une portion déformable 72A, 72B. Cette portion déformable peut par exemple prendre la forme d'un soufflet dont l'extension verticale z (i.e la hauteur) est modifiable. Le moyen de réglage 71 comprend avantageusement ladite portion déformable 72A, 72B ainsi qu'un organe de commande 73 permettant de commander la déformation de ladite portion déformable 72A, 72B pour modifier l'altitude dudit premier et/ou dudit second orifice 63, 64, solidaire(s) de la portion déformable 72A, 72B. L'organe de commande 73 peut par exemple comporter un actionneur mécanique, tel qu'un vérin 74 conçu pour déformer la portion déformable 72A, 72B, par exemple en lui appliquant un effort de traction ou de compression pour augmenter ou diminuer la hauteur z. L'organe de commande 73 comprend avantageusement un moyen pour commander informatiquement ou électroniquement ledit actionneur, ce qui permet un réglage fin et aisé de la position de l'orifice de sortie 63, 64 qu'il contrôle.

Avantageusement, la portion déformable est la partie supérieure du tube de diamètre inférieur 61A, 62A. Ainsi la modification de la hauteur z des orifices de sortie 63, 64 ne modifie pas le système d'évacuation du fluide. Les canaux de séparation 61 et d'écoulement 62 comprennent respectivement une connexion de sortie 63A, 64A, chacune d'elle étant reliée à l'ouverture de sortie 33. Ainsi les connexions de sortie 63A, 64A sont fixes lorsque les orifices de sortie 63, 64 changent d'altitude.

Par ailleurs, l'outil de caractérisation 3 comprend avantageusement un moyen de réglage du débit 85 en fluide multiphasique pénétrant au sein de l'outil d'analyse 3. Ce moyen de réglage 85 permet ainsi de modifier le temps de séjour de l'émulsion au sein du décanteur d'analyse 6. Avantageusement, le moyen de réglage 85 du débit entrant au sein du décanteur d'analyse 6 comprend au moins une pompe volumétrique P1 monté en amont du décanteur d'analyse 6 et en aval de l'ouverture d'entrée 31. En réglant le débit, on peut ainsi modifier le temps de séjour de l'émulsion au sein du décanteur d'analyse 6 et ainsi caractériser son comportement en fonction de ce temps de séjour. Avantageusement, grâce au moyen de réglage 85, il est possible par exemple de régler le débit de façon que les temps de séjour de l'émulsion au sein des décanteurs principal 2 et d'analyse 6 soient identiques, de sorte que l'étude de la séparation au sein du décanteur d'analyse 6 procure des enseignements directement exploitables pour le pilotage du décanteur principal 2, sans avoir à intervenir directement sur ce dernier, ce qui serait en pratique très complexe à mettre en œuvre.

Avantageusement, le moyen de réglage 85 du débit entrant permet également de couper l'alimentation en fluide de l'outil de caractérisation 3. Ceci peut s'avérer intéressant si l'exploitant de l'installation 1 souhaite étudier la séparation du fluide multiphasique circulant au sein du circuit 4 uniquement sur certaines plages horaires, séparées par des intervalles de pause.

De manière préférentielle, le moyen de réglage 85 peut permettre non seulement d'imposer des conditions de séparation similaires entre le décanteur principal 2 et le décanteur d'analyse 6, mais peut également permettre de simuler le fonctionnement du décanteur principal 2 dans une plage de fonctionnement différente, ce qui permet de tester des solutions destinées à optimiser le pilotage du fonctionnement du décanteur principal 2. L'outil de caractérisation 3 selon l'invention est non seulement utile en tant que simulateur du fonctionnement du décanteur principal 2 mais également en tant qu'outil permettant de prédire le fonctionnement du décanteur principal 2 avec des paramètres d'entrée différents. Il est ainsi possible de modifier le débit entrant afin d'observer les conséquences sur la séparation du fluide. Si la séparation est jugée satisfaisante, on pourra dès lors appliquer une telle solution pour le décanteur principal 2. Mais, l'application directe de la solution peut également prendre en compte d'autres paramètres, comme par exemple les différences entre l'émulsion circulant au sein du circuit 4 et celle circulant au sein du décanteur d'analyse 6. Ces différences peuvent notamment provenir de la fragmentation des gouttes lors de la dérivation de la fraction du flux de fluide multiphasique circulant au sein du circuit 4. On peut ainsi optimiser les conditions opératoires en fonction du comportement réel de l'émulsion et des spécifications des phases séparées.

Avantageusement, l'outil de caractérisation 3 comprend une entrée additionnelle permettant l'ajout d'additifs chimiques au sein de l'outil de caractérisation 3, de préférence à l'amont ou directement dans le décanteur d'analyse 6. Ainsi, l'outil de caractérisation 3 permet avantageusement de simuler l'ajout d'additifs au sein du décanteur principal 2. On peut donc, à l'aide de l'outil de caractérisation 3 selon l'invention, jouer en conditions réelles sur les deux leviers principaux (débit, nature et teneur des additifs chimiques) permettant de piloter le décanteur principal 2, sans pour autant intervenir sur le décanteur principal 2 lui-même. L'outil de caractérisation 3 permet ainsi de déterminer le réglage optimal pour le décanteur principal 2, sans risquer de perturber la production

Préférentiellement, l'outil de caractérisation 3 comprend un moyen de localisation, au sein dudit décanteur d'analyse 6, des phases dudit fluide multiphasique et donc de la zone intermédiaire 26, en vue en particulier de connaitre la hauteur h de cette zone intermédiaire 26. Le moyen de localisation en question permet notamment de savoir si cette zone 26 est bien localisée au sein du premier canal de séparation 61 et non pas au moins en partie dans le deuxième canal de séparation, ce qui serait synonyme d'un dysfonctionnement du décanteur d'analyse 6. La mesure de la hauteur h de la zone intermédiaire 26 peut être faite en continu, ce qui permet de s'assurer qu'elle ne grossit pas ou ne risque pas de se retrouver dans le canal d'écoulement 62. En étudiant cette zone 26, on peut récolter des informations particulièrement pertinentes pour la caractérisation de la séparabilité du fluide, notamment en récoltant des informations sur le phénomène de coalescence se produisant au sein de cette zone 26. Cela permet également de prédire d'éventuels futurs dysfonctionnements du décanteur principal 2.

Ledit moyen de localisation est par exemple formé par plusieurs capteurs de pression 84 étagés verticalement au sein du canal de séparation 61. En effet, la composition et/ ou les propriétés physico-chimiques du fluide, notamment sa densité, influent sur la pression induite par la colonne de liquide de sorte qu'une série de mesures de pression effectuées le long du canal 61 permet de localiser les différentes phases, la cartographie des pressions permettant de déduire la cartographie des phases. Selon une autre variante, ledit moyen de localisation peut être formé par des sondes d'absorption de rayonnements y étagées verticalement qui donnent une mesure directe de la densité du fluide en contact avec elles. On peut dès lors aisément réaliser cette cartographie des phases.

Ainsi, comme exposé dans ce qui précède, le décanteur d'analyse 6 permet la séparation du fluide multiphasique, comme cela est notamment illustré au travers de la figure 2. Cette figure représente uniquement le décanteur d'analyse 6, sans le reste de l'installation de séparation 1. Les deux phases sont évacuées par débordement au niveau des deux orifices de sortie 63, 64. Ainsi le fluide porteur (celui constituant majoritairement l'émulsion) circule au sein du canal de séparation 61 avec une certaine vitesse verticale, non nulle mais suffisamment faible pour permettre la séparation des phases. Si la vitesse de décantation d'une goutte de l'une des phases est inférieure à la vitesse du fluide porteur, cette goutte ne pourra pas rejoindre l'orifice de sortie 63, 64 par lequel la phase correspondante est évacuée. Cette situation est représentée sur la figure 2 avec la présence de gouttes d'une phase dans une autre au niveau des deux orifices de sortie 63, 64. Les gouttes de petite taille sont bien entendu plus difficilement séparées car leur vitesse de décantation est faible. L'analyse des différentes sorties huile et eau au cours du temps permet ainsi de mesurer les performances de séparation du décanteur d'analyse 6 et indirectement celles du décanteur principal 2, ou de manière absolue de caractériser la séparabilité gravitaire de l'émulsion.

Différentes mesures sont avantageusement effectuées au niveau de l'une et/ou de l'autre sortie 63, 64 par lesquelles sont respectivement évacuées les différentes phases. Préférentiellement, ces mesures sont effectuées au niveau des deux sorties. A cette fin, l'outil de caractérisation 3 comprend par exemple au moins un appareil 80 mesurant le débit de l'une desdites phases séparées et évacuées au travers de l'un desdits orifices de sortie 63, 64. En première approche, le débit au travers d'une sortie correspondant à une phase est sensiblement égal au débit total multiplié par la proportion de cette phase au sein du fluide multiphasique. Ainsi la mesure du débit d'une phase donnée permet d'accéder à la proportion d'une phase dans une autre. Cette mesure relativement simple à mettre en oeuvre permet de recueillir une information rapide et en direct de la composition de l'émulsion entrante, information évidemment particulièrement important pour l'exploitant d'un site de production pétrolière.

Un tel appareil de mesure 80 est avantageusement positionné à l'aval des canaux de séparation 61 et d'écoulement 62 et en amont de l'ouverture de sortie 33. Il peut mesurer le débit de la phase aqueuse, tandis qu'un autre appareil 80' peut éventuellement mesurer le débit de la phase organique.

De manière préférentielle, l'outil de caractérisation 3 comprend au moins un capteur 81 mesurant la densité de l'une desdites phases séparées et évacuées au travers de l'un desdits orifices de sortie 63, 64, ledit capteur 81 étant disposé entre ledit un desdits orifices de sortie 63, 64 et ladite ouverture de sortie 33. Ce type de mesure permet d'avoir une information sur le fluide circulant au travers de cet orifice de sortie 63, 64 et permet ainsi de très rapidement prévenir tout dysfonctionnement du décanteur principal 2. De préférence, l'outil de caractérisation 3 comprend deux capteurs 81, 81A respectivement positionnés en aval desdits premier et second orifice de sortie 63, 64 et en amont de l'ouverture de sortie 33, afin de faire des mesures sur l'une et/ou l'autre des phases séparés par le décanteur d'analyse 6.

Avantageusement, l'outil de caractérisation 3 comprend en outre au moins un appareil 82 mesurant, dans l'une desdites phases séparée et évacuée au travers de l'un desdits orifices de sortie 63, 64, l'éventuelle quantité résiduelle présente d'une autre phase dudit fluide multiphasique. Ce type de mesure permet d'évaluer les performances du décanteur d'analyse 6 à un débit donné. On mesure ainsi la séparabilité du fluide à un débit donné. On se sert alors de cette série de mesures pour appliquer la solution la plus performante (variation de débit ou ajout d'additifs chimiques) au décanteur principal 2.

Les mesures présentées précédemment sont mis en œuvre sensiblement plus facilement au sein de l'outil d'analyse 6 plutôt que sur les sorties 22A, 22B du décanteur principal 2. En effet, il peut s'avérer particulièrement avantageux, pour caractériser finement et « *in-situ* » le comportement du fluide multiphasique, de faire varier le débit dudit fluide, notamment pour déterminer sa « *séparabilité* » intrinsèque. Or, il n'est pas envisageable de faire varier le débit de production du circuit principal pour réaliser ces mesures, car cela perturberait de façon inacceptable la production. On ne peut donc pas prédire le comportement du fluide multiphasique à l'aide de mesures sur les sorties 22A et 22B. Déporter en dérivation le lieu de mesure, conformément à l'invention, garantit une précision meilleure car l'environnement de l'outil de caractérisation 3 a été précisément conçu pour optimiser cette prise de mesure.

Dans ce qui précède on a décrit un décanteur d'analyse 6 pouvant séparer deux phases liquides et une gazeuse. L'outil de caractérisation 3 selon l'invention permet également d'analyser la séparation d'un liquide triphasique ou comportant encore plus de phases distinctes. Une variante d'un décanteur d'analyse 6 de l'invention destiné à séparer trois phases liquides est représentée sur la figure 3.

Dans le mode de réalisation de la figure 3, le décanteur d'analyse 6 comprend trois canaux de séparation dont chacun est pourvu d'une sortie destinée à évacuer une phase séparée. Ces canaux de séparation s'étendent verticalement entre des bases et des sommets. Toutes les bases de ces canaux sont reliées ensemble par une connexion de raccord 70. De plus, les deux premiers canaux sont reliés également par une connexion supplémentaire de raccord 70', disposée à une altitude supérieure à celle de la connexion de raccord 70, de manière à permettre aux phases du fluide de se répartir au sein des différents canaux de séparation. Les différences de densité entre les phases du fluide multiphasique imposent l'altitude de ce raccord supplémentaire 70'. Avantageusement, l'altitude du raccord 70' peut être modifiée afin que cette variante puisse facilement s'adapter à une large gamme de fluides. Le fonctionnement général est quant à lui analogue à celui d'un outil de caractérisation 3 destiné à la séparation d'un liquide biphasique tel que décrit précédemment. Alternativement, on peut également brancher deux outils de caractérisation 3 en série. Dans cette situation le fluide multiphasique entre dans l'ouverture d'entrée 31 du premier outil de caractérisation 3, la première phase étant séparée et évacuée par le premier orifice de sortie 63, les deux autres phases étant évacuées par le second orifice de sortie 64. Il suffit dès lors de brancher cet orifice de sortie 64 à l'ouverture d'entrée 31 du second outil 3 pour permettre la séparation des deux autres phases.

L'invention concerne également un procédé de caractérisation d'un fluide multiphasique comprenant au moins deux phases liquides de densités respectives différentes, ledit procédé étant avantageusement destiné à être mis en œuvre au moyen de l'installation 1 conforme à la description qui précède. L'ensemble de la description concernant l'installation 1 et son fonctionnement est donc également valable pour le présent procédé. Comme exposé précédemment, ledit fluide est destiné à circuler au sein d'un circuit 4 comprenant un décanteur principal 2 destiné à séparer au moins lesdites phases liquides, ledit décanteur principal 2 comprenant au moins une entrée 21 par laquelle ledit fluide multiphasique est destiné à pénétrer dans le décanteur principal 2 et au moins une sortie 22A, 22B, 22C par laquelle ledit fluide est destiné à être évacué hors du décanteur principal 2. Les caractéristiques physico-chimiques du fluide multiphasique dépendent notamment des conditions amont de production du fluide multiphasique. Avantageusement, le fluide pénètre au sein du décanteur principal 2 selon un flux entrant.

Le procédé selon l'invention comprend, comme exposé précédemment, une étape de dérivation d'une fraction du fluide vers un décanteur d'analyse 6, de façon que le décanteur principal 2 et le décanteur d'analyse 6 soient alimentés concomitamment en fluide multiphasique, ladite fraction formant un flux d'analyse passant par ledit décanteur d'analyse 6, afin de séparer au moins lesdites phases liquides, et ressortant de ce dernier sous la forme de phases séparées. Les phases séparées forment avantageusement un flux analysé. Ainsi, le flux d'analyse représente une portion du flux de fluide circulant au sein du circuit 4 au niveau de la dérivation. Avantageusement, le lieu de la dérivation se situe en amont de ladite entrée 21 dudit décanteur principal 2. Le flux analysé est au sens de l'invention le flux de fluide en sortie du décanteur d'analyse 6. Avantageusement, le décanteur d'analyse 6 permet la séparation desdites phases du fluide et comprend au moins un premier et un second orifice de sortie 63, 64 par lesquels sont respectivement évacuées lesdites phases séparées. Ainsi, le flux analysé est avantageusement formé de deux sous-flux séparés de phases différentes.

De manière préférentielle, le procédé selon l'invention comprend une étape de réglage de débit dudit flux d'analyse circulant au travers dudit décanteur d'analyse 6 en modifiant la quantité de fluide formant ladite fraction. De préférence, cette étape de réglage comprend une étape d'augmentation ou de diminution du débit. Ceci permet notamment de modifier le temps de séjour du fluide au sein du décanteur d'analyse 6. Avantageusement le procédé selon l'invention comprend également une étape d'introduction d'additifs chimiques au sein du décanteur principal 2.

Le procédé selon l'invention comprend également, comme exposé précédemment en relation avec la description de l'installation de séparation 1, une étape d'injection desdites phases séparées au sein dudit circuit 4, en amont de la sortie 22 du décanteur principal 2. L'installation 1 selon l'invention caractérise le fluide multiphasique circulant en son sein, ce qui nécessite la séparation desdites phases. Mais cette séparation n'a pas de conséquences sur la production car les phases séparées sont toutes les deux réinjectées dans le circuit 4. Avantageusement, le fluide circule continûment et simultanément au sein à la fois du décanteur d'analyse 6 et au sein du décanteur principal 2.

Le procédé selon l'invention comprend également une étape de réunion desdites phases séparées. La réunion des ces phases séparées est effectuée de préférence à l'aval du décanteur d'analyse 6 par rapport au sens d'écoulement de ladite fraction au sein dudit outil 3. Comme exposé précédemment, cette réunion peut avoir lieu en amont de l'ouverture de sortie 33 ou directement au sein du circuit 4 si les phases séparées sont directement injectées au sein du circuit 4. Ainsi, l'étape de réunion peut s'effectuer avant ou après l'étape d'injection.

De manière préférentielle, le procédé d'analyse selon l'invention comprend une étape de mesure de la densité de l'une desdites phases séparée permettant notamment de s'assurer de tout dysfonctionnement majeur du décanteur d'analyse 6.

Avantageusement, le procédé d'analyse selon l'invention comprend une étape de mesure, dans au moins l'une desdites phases séparée, de l'éventuelle quantité résiduelle présente d'une autre phase dudit fluide. Cette mesure peut être effectuée par tout moyen connu, comme par exemple à l'aide de capteurs capacitifs ou optiques. Cette information s'avère utile pour accéder aux performances de séparation du décanteur d'analyse 6 et ainsi également par déduction à celles du décanteur principal 2.

Préférentiellement, le procédé d'analyse selon l'invention comprend une étape de mesure du débit de l'une desdites phases séparée. Comme explicitée précédemment, cela permet notamment de connaître les proportions respectives des phases au sein du fluide multiphasique.

Avantageusement, le procédé selon l'invention permet, à partir des étapes décrites précédemment, de caractériser la séparabilité du fluide multiphasique circulant au sein du circuit 4. Pour cela, le procédé comprend avantageusement les étapes successives suivantes :
- une étape de réglage du flux d'analyse à un premier débit de mesure. Le débit de mesure peut ainsi prendre des valeurs arbitrairement fortes ou faibles.
- une étape de mesure dans l'une desdites phases séparées de l'éventuelle quantité résiduelle présente d'une autre phase dudit fluide donnant lieu à la détermination d'une valeur de mesure correspondant audit premier débit de mesure. Avantageusement, on réalise cette mesure de teneur résiduelle sur les deux sorties 63, 64 du décanteur d'analyse 6 afin d'avoir une mesure particulièrement précise.
- une étape de sauvegarde de ladite valeur de mesure en fonction dudit débit de mesure. Les valeurs mesurées sont ainsi stockées afin de pouvoir être facilement transmises et analysées.

Les étapes de réglage du flux, mesure et sauvegarde sont répétées à des débits de mesure différents afin de caractériser la séparation dudit fluide en fonction du débit de mesure. Avantageusement, on peut construire une courbe de la teneur résiduelle d'une phase dans une autre en fonction du débit entrant ou du temps de séjour de l'émulsion au sein du décanteur d'analyse 6, le temps de séjour et le débit étant proportionnels. On dispose ainsi d'une information particulièrement pertinente pour caractériser l'émulsion. De manière préférentielle, cette information se révèle utile pour piloter le fonctionnement du décanteur principal 2 à un débit maximal pour une teneur résiduelle d'une phase dans une autre considérée comme acceptable.

De manière préférentielle, le procédé d'analyse selon l'invention comprend une étape de localisation, au sein dudit décanteur d'analyse 6, des phases dudit fluide multiphasique. Cela peut être réalisé à l'aide de capteurs de pression 84. Cette étape de localisation comprend avantageusement les sous étapes suivantes :
- une sous-étape de mesure de pression. Cette sous-étape peut par exemple être effectuée à l'aide d'une série de capteur de pression 84 étagés verticalement, afin de cartographier le champ de pression selon une direction verticale. Elle peut également être effectuée à l'aide de sondes d'absorption de rayonnements y, comme exposé précédemment.
- une sous-étape d'identification de l'étendue verticale des différentes phases du fluide, par détermination des variations brusques du champ de pression champ de pression, afin de déterminer l'altitude des différentes zones 27, 25, 26 précitées.

L'étape de localisation comprend avantageusement une sous-étape de mesure de la hauteur h de la zone intermédiaire 26.

Le procédé selon l'invention permet avantageusement de simuler le fonctionnement du décanteur principal 2. En modifiant le débit d'entrée et/ou en ajoutant des additifs chimiques, on peut simuler des conditions spécifiques pouvant être appliquées au décanteur principal 2. Pour cela, on modifie par exemple le débit du flux d'analyse et/ou la nature et la teneur des additifs chimiques. Ensuite on mesure la qualité de la séparation du fluide au sein du décanteur d'analyse 6, à l'aide notamment de mesures de densité, de débit, ou de proportion de phases dans une autre, comme exposé précédemment. Cela permet de définir les performances pour les conditions du test. Si elles sont acceptables, elles peuvent être appliquées au décanteur principal 2. On évite ainsi de tester des solutions directement sur le décanteur principal 2, limitant ainsi le risque de graves dysfonctionnements, voire même d'interruption de l'unité de production.

Ainsi, le procédé selon l'invention permet de caractériser les propriétés de séparation d'une émulsion donnée. De manière préférentielle, le procédé selon l'invention permet plus précisément de caractériser la séparabilité gravitaire de l'émulsion. Lorsque l'installation de traitement 1 comprend un décanteur principal 2, le procédé selon l'invention peut être avantageusement mis en œuvre selon deux modes alternatifs de fonctionnement : un mode de routine dans lequel le décanteur d'analyse 6 a des conditions de fonctionnement similaires à celles du décanteur principal 2 et un mode d'anticipation, selon lequel le décanteur d'analyse 6 permet de tester des solutions pour le pilotage du décanteur principal 2 sans pour autant modifier le fonctionnement du décanteur principal 2. Bien évidemment, l'outil de caractérisation 3 peut également être arrêté, aucun fluide ne circulant alors en son sein, sans que cela n'impacte le fonctionnement du décanteur principal 2.

L'installation de séparation 1 et le procédé permettent donc de déterminer la séparabilité gravitaire de chaque phase présentes au sein d'un fluide multiphasique afin notamment de donner des indications pour améliorer le fonctionnement d'un décanteur principal 2, ce qui garantit un gain important de productivité du site industriel dans lequel il est implanté.

### POSSIBILITE D'APPLICATION INDUSTRIELLE

L'invention trouve son application industrielle dans la conception, la fabrication et la mise en œuvre d'installations et de procédé de traitement de fluide multiphasique.

## Revendications

1. Installation de traitement (1) d'un fluide multiphasique comprenant au moins une émulsion de deux phases liquides de densités respectives différentes, ladite installation (1) comprenant un circuit (4) au sein duquel est destiné à circuler ledit fluide multiphasique, ledit circuit (4) comprenant un décanteur principal (2) destiné à séparer au moins lesdites phases liquides, ledit décanteur principal (2) comprenant au moins une entrée (21) par laquelle ledit fluide multiphasique est destiné à pénétrer dans le décanteur principal (2) et au moins une sortie (22A, 22B, 22C) par laquelle ledit fluide est destiné à être évacué hors du décanteur principal (2), ladite installation étant **caractérisée en ce qu'**elle comprend également un outil de caractérisation (3) dudit fluide multiphasique, ledit outil de caractérisation (3) comprenant au moins :
- une ouverture d'entrée (31) et une ouverture de sortie (33) respectivement connectées audit circuit (4) en amont de ladite entrée (21) et en amont de ladite sortie (22A, 22B, 22C) relativement au sens d'écoulement dudit fluide multiphasique au sein du circuit (4),
- un décanteur d'analyse (6) disposé entre lesdites ouvertures d'entrée (31) et de sortie (33) et apte à séparer au moins lesdites phases liquides pour obtenir des phases dites séparées,
- un moyen de réunion (9) desdites phases séparées,
ladite installation (1) étant conçue pour dériver une fraction du flux de fluide multiphasique alimentant le décanteur principal (2) vers et dans l'outil de caractérisation (3), de façon que le décanteur principal (2) et l'outil de caractérisation (3) soient alimentés concomitamment en fluide multiphasique, le fluide multiphasique pénétrant au sein de l'outil de caractérisation (3) étant donc sensiblement similaire au fluide multiphasique pénétrant au sein du décanteur principal (2), ladite fraction du fluide circulant au sein de l'outil (3) en pénétrant par ladite ouverture d'entrée (31) pour passer par ledit décanteur d'analyse (6) afin que lesdites phases soient séparées puis évacuées par ladite ouverture de sortie (33) dans ledit circuit (4), la phase gazeuse présente au sein de l'émulsion étant au moins partiellement pré-séparée dans l'outil de caractérisation (3) avant que le fluide multiphasique ne pénètre dans le décanteur d'analyse (6), ledit moyen de réunion (9) étant conçu pour réunir lesdites phases séparées à l'aval du décanteur d'analyse (6) et en amont de ladite sortie (22A, 22B, 22C) par rapport au sens d'écoulement du fluide.

2. Installation de traitement (1) d'un fluide multiphasique selon la revendication 1 **caractérisée en ce qu'**elle constitue soit un élément d'une unité de production pétrolière, ledit fluide comprenant du pétrole, soit un élément d'une station d'épuration, ledit fluide comprenant des eaux altérées par des activités humaines à la suite d'un usage domestique, industriel ou agricole.

3. Installation de traitement (1) d'un fluide multiphasique selon l'une des revendications précédentes **caractérisée en ce que** ledit outil de caractérisation (3) comprend un moyen de limitation du débit (83) en fluide multiphasique pénétrant au sein dudit outil (3), de façon que le débit de fluide multiphasique entrant au sein dudit outil de caractérisation (3) soit inférieur au débit de fluide multiphasique circulant dans ledit circuit (4) au niveau de ladite ouverture d'entrée (31).

4. Installation de traitement (1) d'un fluide multiphasique selon l'une des revendications précédentes **caractérisée en ce que** ledit outil de caractérisation (3) comprend un moyen de réglage du débit (85) en fluide multiphasique pénétrant au sein dudit outil (3).

5. Installation de traitement (1) d'un fluide multiphasique selon l'une des revendications précédentes **caractérisée en ce que** ledit décanteur d'analyse (6) comprend au moins un canal de séparation (61) pourvu d'un premier orifice de sortie (63) et s'étendant verticalement entre une première base (65) et un premier sommet (66) et un canal d'écoulement (62) pourvu d'un second orifice de sortie (64) et s'étendant verticalement entre une seconde base (67) et un second sommet (68), ledit décanteur d'analyse (6) comprenant une connexion de raccord (70) connectant lesdits premier et second canal de séparation (61) et d'écoulement (62) vers leurs bases (65, 67), lesdits orifices de sortie (63, 64) étant reliés à ladite ouverture de sortie (33), ledit canal de séparation (61) étant connecté à ladite ouverture d'entrée (31) pour alimenter ledit décanteur d'analyse (6) en fluide, lesdits orifices de sortie (63, 64) étant étagés verticalement de façon à séparer lesdites phases du fluide, chaque phase ainsi séparée étant ensuite évacuée au travers de l'un desdits orifices de sortie (63, 64).

6. Installation de traitement (1) d'un fluide multiphasique selon la revendication précédente **caractérisée en ce que** ledit outil comprend un moyen de réglage (71) de la position verticale du premier et/ou du second orifice de sortie (63, 64).

7. Installation de traitement (1) d'un fluide multiphasique selon la revendication précédente **caractérisée en ce que** ledit canal de séparation (61) et/ou ledit canal d'écoulement (62) comprend (comprennent) une portion déformable (72A, 72B) et **en ce que** ledit moyen de réglage (71) comprend ladite portion déformable (72A, 72B) ainsi qu'un organe de commande (73) permettant de commander la déformation de ladite portion déformable (72A, 72B) pour modifier l'altitude dudit premier et/ou dudit second orifice (63, 64).

8. Installation de traitement (1) d'un fluide multiphasique selon l'une des revendications 5 à 7 **caractérisée en ce que** ledit outil (3) comprend au moins un appareil (80) mesurant le débit de l'une desdites phases séparée et évacuée au travers de l'un desdits orifices de sortie (63, 64) et/ou au moins un appareil (82) mesurant, dans l'une desdites phases séparée et évacuée au travers de l'un desdits orifices de sortie (63, 64), l'éventuelle quantité résiduelle présente d'une autre phase dudit fluide multiphasique.

9. Installation de traitement (1) d'un fluide multiphasique selon l'une des revendications précédentes **caractérisée en ce que** ledit outil (3) comprend un moyen de localisation au sein dudit décanteur d'analyse (6) des phases dudit fluide multiphasique.

10. Installation de traitement (1) d'un fluide multiphasique selon l'une des revendications précédentes **caractérisée en ce que** ledit décanteur principal (2) et ledit décanteur d'analyse (6) comprennent chacun respectivement une première et une seconde chambre de séparation (23, 60) au sein desquelles ledit fluide est destiné à circuler, le volume de ladite première chambre de séparation (23) étant au moins dix fois plus grand que le volume de ladite seconde chambre de séparation (60), préférentiellement au moins cent fois plus grand.

11. Installation de traitement (1) d'un fluide multiphasique selon l'une des deux revendications précédentes **caractérisée en ce que** ledit circuit (4) comprend au moins un conduit d'acheminement (5) de fluide pourvu d'un embranchement (51) divisant ledit conduit en au moins une première et une deuxième branche (52, 53), ladite première branche (52) étant connectée à ladite entrée (21) et ladite deuxième branche (53) étant connectée à ladite ouverture d'entrée (31) pour permettre l'alimentation concomitante en fluide dudit décanteur principal (2) et dudit outil (3).

12. Installation de traitement (1) d'un fluide multiphasique selon la revendication précédente **caractérisée en ce que** ladite ouverture de sortie (33) est connectée audit conduit (5) et/ou audit décanteur principal (2).

13. Procédé de caractérisation d'un fluide multiphasique comprenant au moins une émulsion de deux phases liquides de densités respectives différentes, ledit fluide étant destiné à circuler au sein d'un circuit (4) comprenant un décanteur principal (2) destiné à séparer au moins lesdites phases liquides, ledit décanteur principal (2) comprenant au moins une entrée (21) par laquelle ledit fluide multiphasique est destiné à pénétrer dans le décanteur principal (2) et au moins une sortie (22A, 22B, 22C) par laquelle ledit fluide est destiné à être évacué hors du décanteur principal (2), ledit procédé étant **caractérisé en ce qu'**il comprend les étapes suivantes :
- une étape de dérivation d'une fraction du flux de fluide alimentant le décanteur principal (2) vers un décanteur d'analyse (6), de façon que le décanteur principal (2) et le décanteur d'analyse (6) soient alimentés concomitamment en fluide multiphasique, le fluide multiphasique pénétrant au sein dudit décanteur d'analyse (6) étant donc sensiblement similaire au fluide multiphasique pénétrant au sein du décanteur principal (2), ladite fraction formant un flux d'analyse passant par ledit décanteur d'analyse (6), afin de séparer au moins lesdites phases liquides, et ressortant de ce dernier sous la forme de phases séparées, la phase gazeuse présente au sein de l'émulsion étant au moins partiellement pré-séparée dans l'outil de caractérisation (3) avant que le fluide multiphasique ne pénètre dans le décanteur d'analyse (6)
- une étape de réunion desdites phases séparées,
- une étape d'injection desdites phases séparées au sein dudit circuit (4) en amont de la sortie (22A, 22B, 22C) du décanteur principal (2).

14. Procédé de caractérisation d'un fluide multiphasique selon la revendication précédente **caractérisé en ce qu'**il comprend une étape de réglage de débit dudit flux d'analyse circulant au travers dudit décanteur d'analyse (6) en modifiant la quantité de fluide formant ladite fraction.

15. Procédé de caractérisation d'un fluide multiphasique selon la revendication précédente **caractérisé en ce qu'**il comprend une étape de mesure, dans au moins l'une desdites phases séparée, de l'éventuelle quantité résiduelle présente d'une autre phase dudit fluide, ainsi que les étapes successives suivantes :
- une étape de réglage du flux d'analyse à un premier débit de mesure,
- une étape de mesure dans l'une desdites phases séparées de l'éventuelle quantité résiduelle présente d'une autre phase dudit fluide donnant lieu à la détermination d'une valeur de mesure correspondant audit premier débit de mesure,
- une étape de sauvegarde de ladite valeur de mesure en fonction dudit débit de mesure,
lesdites étapes de réglage du flux, mesure et sauvegarde étant répétées à des débits de mesure différents afin de caractériser la séparation dudit fluide en fonction du débit de mesure.

16. Procédé de caractérisation d'un fluide multiphasique selon l'une des revendications 13 à 15 **caractérisé en ce que** ledit procédé comprend une étape de localisation, au sein dudit décanteur d'analyse (6), des phases dudit fluide multiphasique et/ou une étape de mesure du débit de l'une desdites phases séparées.

## Patentansprüche

1. Anlage (1) zur Verarbeitung eines mehrphasigen Fluids, umfassend mindestens eine Emulsion von zwei Flüssigphasen mit jeweils unterschiedlicher Dichte, wobei die genannte Anlage (1) einen Kreislauf (4) umfasst, in dem das genannte mehrphasige Fluid dazu bestimmt ist zu zirkulieren, wobei der genannte Kreislauf (4) einen Hauptdekanter (2) umfasst, der dazu bestimmt ist, mindestens die genannten Flüssigphasen zu trennen, wobei der genannte Hauptdekanter (2) mindestens einen Einlass (21), durch den das genannte mehrphasige Fluid dazu bestimmt ist, in den Hauptdekanter (2) einzudringen, und mindestens einen Auslass (22A, 22B, 22C) umfasst, durch den das genannte Fluid dazu bestimmt ist, aus dem Hauptdekanter (2) abgeführt zu werden, wobei die genannte Anlage **dadurch gekennzeichnet ist, dass** sie auch ein Charakterisierungswerkzeug (3) des genannten mehrphasigen Fluids umfasst, wobei das genannte Charakterisierungswerkzeug (3) mindestens umfasst:
- eine Einlassöffnung (31) und eine Auslassöffnung (33), die jeweils mit dem genannten Kreislauf (4) stromaufwärts von dem genannten Einlass (21) und stromaufwärts von dem genannten Auslass (22A, 22B, 22C) relativ in der Strömungsrichtung des genannten mehrphasigen Fluids in dem Kreislauf (4) verbunden sind,
- einen Analysedekanter (6), der zwischen der genannten Einlassöffnung (31) und Auslassöffnung (33) angeordnet ist und geeignet ist, mindestens die genannten Flüssigphasen zu trennen, um sogenannte getrennte Phasen zu erhalten,
- ein Vereinigungsmittel (9) der genannten getrennten Phasen,
wobei die genannte Anlage (1) ausgelegt ist, um eine Fraktion des Stroms des mehrphasigen Fluids, das den Hauptdekanter (2) versorgt, zu dem und in das Charakterisierungswerkzeug (3) zu leiten, damit der Hauptdekanter (2) und das Charakterisierungswerkzeug (3) gleichzeitig mit dem mehrphasigen Fluid versorgt werden, wobei das mehrphasige Fluid in das Innere des Charakterisierungswerkzeugs (3) eindringt, das somit im Wesentlichen ähnlich dem mehrphasigen Fluid ist, das in das Innere des Hauptdekanters (2) eindringt, wobei die genannte Fraktion des Fluids, die im Inneren des Werkzeugs (3) zirkuliert, durch die genannte Einlassöffnung (31) eindringt, um durch den genannten Analysedekanter (6) hindurchzugehen, damit die genannten Phasen getrennt werden und dann durch die genannte Auslassöffnung (33) in den genannten Kreislauf (4) abgeführt werden, wobei die Gasphase, die in der Emulsion vorhanden ist, mindestens teilweise in dem Charakterisierungswerkzeug (3) vorgetrennt wird, bevor das mehrphasige Fluid in den Analysedekanter (6) eindringt, wobei das Vereinigungsmittel (9) ausgelegt ist, um die genannten getrennten Phasen stromabwärts von dem Analysedekanter (6) und stromaufwärts von dem genannten Auslass (22A, 22B, 22C) in Bezug auf die Strömungsrichtung des Fluids zu vereinigen.

2. Anlage (1) zur Verarbeitung eines mehrphasigen Fluids nach Anspruch 1, **dadurch gekennzeichnet, dass** sie entweder ein Element einer Erdölproduktionseinheit, wobei das genannte Fluid Erdöl umfasst, oder ein Element einer Kläranlage darstellt, wobei das genannte Fluid Wasser umfasst, das durch menschliche Aktivitäten nach einer Verwendung im Haushalt, in der Industrie oder in der Landwirtschaft verändert wurde.

3. Anlage (1) zur Verarbeitung eines mehrphasigen Fluids nach einem der vorhergehenden Ansprüche, **dadurch gekennzeichnet, dass** das genannte Charakterisierungswerkzeug (3) ein Mittel (83) zur Begrenzung des Durchsatzes des mehrphasigen Fluids umfasst, das in das Innere des genannten Werkzeugs (3) eindringt, damit der Durchsatz des mehrphasigen Fluids, das in das Innere des genannten Werkzeugs (3) eindringt, kleiner ist als der Durchsatz des mehrphasigen Fluids, das in dem genannten Kreislauf (4) auf der Höhe der genannten Einlassöffnung (31) zirkuliert.

4. Anlage (1) zur Verarbeitung eines mehrphasigen Fluids nach einem der vorhergehenden Ansprüche, **dadurch gekennzeichnet, dass** das genannte Charakterisierungswerkzeug (3) ein Mittel (85) zum Regeln des Durchsatzes des mehrphasigen Fluids umfasst, das in das Innere des genannten Werkzeugs (3) eindringt.

5. Anlage (1) zur Verarbeitung eines mehrphasigen Fluids nach einem der vorhergehenden Ansprüche, **dadurch gekennzeichnet, dass** der genannte Analysedekanter (6) mindestens einen Trennkanal (61), der mit einer ersten Auslassöffnung (63) versehen ist und sich vertikal zwischen einer ersten Basis (65) und einer ersten Oberseite (66) erstreckt, und einen Strömungskanal (62), der mit einer zweiten Auslassöffnung (64) versehen ist und sich vertikal zwischen einer zweiten Basis (67) und einer zweiten Oberseite (68) erstreckt, umfasst, wobei der genannte Analysedekanter (6) einen Verbindungsanschluss (70) umfasst, der den genannten ersten Trennkanal (61) und zweiten Strömungskanal (62) mit ihren Basen (65, 67) verbindet, wobei die genannten Auslassöffnungen (63, 64) mit der Auslassöffnung (33) verbunden sind, wobei der Trennkanal (61) mit der genannten Einlassöffnung (31) verbunden ist, um den genannten Analysedekanter (6) mit Fluid zu versorgen, wobei die genannten Auslassöffnungen (63, 64) vertikal abgestuft sind, um die genannten Phasen des Fluids zu trennen, wobei jede so getrennte Phase anschließend durch eine der genannten Auslassöffnungen (63, 64) abgeführt wird.

6. Anlage (1) zur Verarbeitung eines mehrphasigen Fluids nach dem vorhergehenden Anspruch, **dadurch gekennzeichnet, dass** das genannte Werkzeug ein Mittel (71) zum Regeln der vertikalen Position der ersten und/oder der zweiten Auslassöffnung (63, 64) umfasst.

7. Anlage (1) zur Verarbeitung eines mehrphasigen Fluids nach dem vorhergehenden Anspruch, **dadurch gekennzeichnet, dass** der genannte Trennkanal (61) und/oder der genannte Strömungskanal (62) einen verformbaren Abschnitt (72A, 72B) umfasst (umfassen), und dadurch, dass das genannte Mittel (71) zum Regeln den genannten verformbaren Abschnitt (72A, 72B) sowie ein Steuerorgan (73) umfasst, das es gestattet, die Verformung des genannten verformbaren Abschnitts (72A, 72B) zu steuern, um die Höhe der genannten ersten und/oder der genannten zweiten Öffnung (63, 64) zu modifizieren.

8. Anlage (1) zur Verarbeitung eines mehrphasigen Fluids nach einem der Ansprüche 5 bis 7, **dadurch gekennzeichnet, dass** das genannte Werkzeug (3) mindestens eine Vorrichtung (80), welche den Durchsatz einer der genannten getrennten und durch eine der genannten Auslassöffnungen (63, 64) abgeführten Phasen misst, und mindestens eine Vorrichtung (82) umfasst, welche, in einer der genannten getrennten und durch eine der genannten Auslassöffnungen (63, 64) abgeführten Phasen, die eventuell vorhandene Restmenge einer anderen Phase des genannten mehrphasigen Fluids misst.

9. Anlage (1) zur Verarbeitung eines mehrphasigen Fluids nach einem der vorhergehenden Ansprüche, **dadurch gekennzeichnet, dass** das genannte Werkzeug (3) ein Mittel zum Lokalisieren von Phasen des genannten mehrphasigen Fluids im Inneren des genannten Analysedekanters (6) umfasst.

10. Anlage (1) zur Verarbeitung eines mehrphasigen Fluids nach einem der vorhergehenden Ansprüche, **dadurch gekennzeichnet, dass** der genannte Hauptdekanter (2) und der genannte Analysedekanter (6) jeder jeweils eine erste und eine zweite Trennkammer (23, 60) umfassen, in deren Innerem das genannte Fluid dazu bestimmt ist zu zirkulieren, wobei das Volumen der genannten ersten Trennkammer (23) mindestens zweimal größer ist als das Volumen der genannten zweiten Trennkammer (60), vorzugsweise mindestens zehnmal größer.

11. Anlage (1) zur Verarbeitung eines mehrphasigen Fluids nach einem der beiden vorhergehenden Ansprüche, **dadurch gekennzeichnet, dass** der genannte Kreislauf (4) mindestens eine Zufuhrleitung (5) des Fluids umfasst, die mit einer Abzweigung (51) versehen ist, welche die genannte Leitung in mindestens einen ersten und einen zweiten Zweig (52, 53) trennt, wobei der genannte erste Zweig (52) mit dem genannten Einlass (21) verbunden ist, und der genannte zweite Zweig (53) mit der genannten Einlassöffnung (31) verbunden ist, um die gleichzeitige Versorgung des genannten Hauptdekanters (2) und des genannten Werkzeugs (3) mit Fluid zu erlauben.

12. Anlage (1) zur Verarbeitung eines mehrphasigen Fluids nach dem vorhergehenden Anspruch, **dadurch gekennzeichnet, dass** die genannte Auslassöffnung (33) mit der genannten Leitung (5) und/oder dem Hauptdekanter (2) verbunden ist.

13. Verfahren zur Charakterisierung eines mehrphasigen Fluids, umfassend mindestens eine Emulsion von zwei Flüssigphasen mit jeweils unterschiedlicher Dichte, wobei das genannte Fluid dazu bestimmt ist, in einer Leitung (4) zu zirkulieren, umfassend einen Hauptdekanter (2), der dazu bestimmt ist, mindestens die genannten Flüssigphasen zu trennen, wobei der genannte Hauptdekanter (2) mindestens einen Einlass (21), durch den das genannte mehrphasige Fluid dazu bestimmt ist, in den Hauptdekanter (2) einzudringen, und mindestens einen Auslass (22A, 22B, 22C) umfasst, durch den das genannte Fluid dazu bestimmt ist, aus dem Hauptdekanter (2) abgeführt zu werden, wobei das genannte Verfahren **dadurch gekennzeichnet ist, dass** es die folgenden Schritte umfasst:
- einen Schritt des Leitens einer Fraktion des Fluidstroms, der den Hauptdekanter (2) versorgt, zu einem Analysedekanter (6), damit der Hauptdekanter (2) und der Analysedekanter (6) gleichzeitig mit dem mehrphasigen Fluid versorgt werden, wobei das mehrphasige Fluid, das in das Innere des genannten Analysedekanters (6) eindringt, somit im Wesentlichen ähnlich ist dem mehrphasigen Fluid, das in das Innere des Hauptdekanters (2) eindringt, wobei die genannte Fraktion einen Analysestrom bildet, der durch den genannten Analysedekanter (6) hindurchgeht, um mindestens die genannten Flüssigphasen zu trennen, und aus diesem Letzteren in der Form getrennter Phasen austritt, wobei die Gasphase, die in der Emulsion vorhanden ist, mindestens teilweise in dem Charakterisierungswerkzeug (3) vorgetrennt wird, bevor das mehrphasige Fluid in den Analysedekanter (6) eindringt,
- einen Schritt des Vereinigens der genannten getrennten Phasen,
- einen Schritt des Injizierens der genannten getrennten Phasen in die genannte Leitung (4) stromaufwärts von dem Auslass (22A, 22B, 22C) des Hauptdekanters (2).

14. Verfahren zur Charakterisierung eines mehrphasigen Fluids nach dem vorhergehenden Anspruch, **dadurch gekennzeichnet, dass** es einen Schritt des Regelns des Durchsatzes des genannten Analysestroms umfasst, der durch den Analysedekanter (6) zirkuliert, indem die Menge des Fluids modifiziert wird, das die Fraktion bildet.

15. Verfahren zur Charakterisierung eines mehrphasigen Fluids nach dem vorhergehenden Anspruch, **dadurch gekennzeichnet, dass** es einen Schritt des Messens, in mindestens einer der genannten getrennten Phasen, der eventuell vorhandenen Restmenge einer anderen Phase des genannten Fluids sowie die folgenden aufeinanderfolgenden Schritte umfasst:
- einen Schritt des Regelns des Analysestroms mit einem ersten Messdurchsatz,
- einen Schritt des Messens, in einer der genannten getrennten Phasen, der eventuell vorhandenen Restmenge einer anderen Phase des genannten Fluids, welcher zur Bestimmung eines Messwerts führt, der dem genannten ersten Messdurchsatz entspricht,
- einen Schritt des Sicherns des genannten Messwerts als Funktion des genannten Messdurchsatzes,
wobei die genannten Schritte des Regelns des Stroms, des Messens und des Sicherns mit unterschiedlichen Messdurchsätzen wiederholt werden, um die Trennung des genannten Fluids als Funktion des Messdurchsatzes zu charakterisieren.

16. Verfahren zur Charakterisierung eines mehrphasigen Fluids nach einem der Ansprüche 13 bis 15, **dadurch gekennzeichnet, dass** das genannte Verfahren einen Schritt des Lokalisierens, im Inneren des Analysedekanters (6), der Phasen des genannten mehrphasigen Fluids und/oder einen Schritt des Messens des Durchsatzes einer der genannten getrennten Phasen umfasst.

## Claims

1. Plant (1) for the treatment of a multiphasic fluid comprising at least an emulsion of two liquid phases of different respective densities, said plant (1) comprising a circuit (4) within which said multiphasic fluid is intended to circulate, said circuit (4) comprising a main settler (2) intended to separate at least said liquid phases, said main settler (2) comprising at least one inlet (21) through which said multiphasic fluid is intended to enter into the main settler (2) and at least one outlet (22A, 22B, 22C) through which said fluid is intended to be discharged from the main settler (2), said plant being **characterized in that** it also comprises a tool (3) for the characterization of said multiphasic fluid, said characterization tool (3) comprising at least:
- one inlet opening (31) and one outlet opening (33) respectively connected to said circuit (4) upstream of said inlet (21) and upstream of said outlet (22A, 22B, 22C) with respect to the flowing direction of said multiphasic fluid within the circuit (4),
- an analysis settler (6) arranged between said inlet (31) and outlet (33) openings and adapted to separate at least said liquid phases to obtain so-called separated phases,
- a means (9) for combining said separated phases,
said plant (1) being designed to divert a fraction of the flow of multiphasic fluid feeding the main settler (2) towards and into the characterization tool (3), so that the main settler (2) and the characterization tool (3) are concomitantly fed with multiphasic fluid, the multiphasic fluid entering into the characterization tool (3) being hence substantially similar to the multiphasic fluid entering into the main settler (2), wherein said fraction of the fluid circulates within the tool (3) entering through said inlet opening (31) to pass through said analysis settler (6) so that said phases are separated then discharged through said outlet opening (33) into said circuit (4), the gaseous phase present within the emulsion being at least partially pre-separated in the characterization tool (3), before the multiphasic fluid enters into the analysis settler (6), said combining means (9) being designed to combine said separated phases downstream of said analysis settler (6) and upstream of said outlet (22A, 22B, 22C) with respect to the flowing direction of the fluid.

2. Plant (1) for the treatment of a multiphasic fluid according to claim 1, **characterized in that** it constitutes either one element of a petroleum production unit, said fluid comprising petroleum, or one element of a sewage treatment plant, said fluid comprising waters altered by human activities, after a domestic, industrial or agricultural use.

3. Plant (1) for the treatment of a multiphasic fluid according to one of the preceding claims, **characterized in that** said characterization tool (3) comprises a means (83) for limiting the flow rate of multiphasic fluid entering into said tool (3), so that the flow rate of multiphasic fluid entering into said characterization tool (3) is lower than the flow rate of the multiphasic fluid circulating in said circuit (4) at said inlet opening (31).

4. Plant (1) for the treatment of a multiphasic fluid according to one of the preceding claims, **characterized in that** said characterization tool (3) comprises a means (85) for adjusting the flow rate of multiphasic fluid entering into said tool (3).

5. Plant (1) for the treatment of a multiphasic fluid according to one of the preceding claims, **characterized in that** said analysis settler (6) comprises at least one separation channel (61) provided with a first outlet orifice (63) and extending vertically between a first base (65) and a first apex (66) and a flowing channel (62) provided with a second outlet orifice (64) and extending vertically between a second base (67) and a second apex (68), said analysis settler (6) comprising a coupling connection (70) connecting said first and second separation (61) and flowing (62) channels towards their bases (65, 67), said outlet orifices (63, 64) being connected to said outlet opening (33), said separation channel (61) being connected to said inlet opening (31) to feed said analysis settler (6) with fluid, said outlet orifices (63, 64) being vertically distributed so as to separate said phases of the fluid, each phase hence separated being then discharged through one of said outlet orifices (63, 64).

6. Plant (1) for the treatment of a multiphasic fluid according to the preceding claim, **characterized in that** said tool comprises a means (71) for adjusting the vertical position of the first and/or second outlet orifice (63, 64).

7. Plant (1) for the treatment of a multiphasic fluid according to the preceding claim, **characterized in that** said separation channel (61) and/or said flowing channel (62) comprises (comprise) a deformable portion (72A, 72B) and **in that** said adjustment means (71) comprises said deformable portion (72A, 72B) as well as a control member (73) allowing to control the deformation of said deformable portion (72A, 72B) to modify the altitude of said first and/or said second orifice (63, 64).

8. Plant (1) for the treatment of a multiphasic fluid according to one of claims 5 to 7, **characterized in that** said tool (3) comprises at least one apparatus (80) measuring the flow rate of one of said phases separated and discharged through one of said outlet orifice (63, 64) and/or at least one apparatus (82) measuring, in one of said phases separated and discharged through one of said outlet orifices (63, 64), the possibly present residual quantity of another phase of said multiphasic fluid.

9. Plant (1) for the treatment of a multiphasic fluid according to one of the preceding claims, **characterized in that** said tool (3) comprises a means for locating within said analysis settler (6) phases of sais multiphasic fluid.

10. Plant (1) for the treatment of a multiphasic fluid according to one of the preceding claims, **characterized in that** said main settler (2) and said analysis settler (6) each comprise a first and a second separation chamber (23, 60), respectively, within which said fluid is intended to circulate, the volume of said first separation chamber (23) being at least ten times greater than the volume of said second separation chamber (60), preferentially at least one hundred time greater.

11. Plant (1) for the treatment of a multiphasic fluid according to one of the two preceding claims, **characterized in that** said circuit (4) comprises at least one fluid supplying duct (5) provided with a fork (51) dividing said duct into at least one first and one second branches (52, 53), said first branch (52) being connected to said inlet (21) and said second branch (53) being connected to said inlet opening (31) to allow the concomitant fluid supply of said main settler (2) and said tool (3).

12. Plant (1) for the treatment of a multiphasic fluid according to one of the preceding claims, **characterized in that** said outlet opening (33) is connected to said duct (5) and/or said main settler (2).

13. Method for characterizing a multiphasic fluid comprising at least an emulsion of two liquid phases of different respective densities, said fluid being intended to circulate within a circuit (4) comprising a main settler (2) intended to separate at least said liquid phases, said main settler (2) comprising at least one inlet (21) through which said multiphasic fluid is intended to enter into the main settler (2) and at least one outlet (22A, 22B, 22C) through which said fluid is intended to be discharged from the main settler (2), said method being **characterized in that** it comprises the following steps:
- a step of diverting a fraction of the flow of fluid feeding the main settler (2) towards an analysis settler (6), so that the main settler (2) and the analysis settler (6) are concomitantly fed with multiphasic fluid, the multiphasic fluid entering into the characterization tool (3) being hence substantially similar to the multiphasic fluid entering into the main settler (2), said fraction forming an analysis flow passing through said analysis settler (6), so as to separate at least said liquid phases, and flowing from the latter as separated phases, the gaseous phase present within the emulsion being at least partially pre-separated in the characterization tool (3), before the multiphasic fluid enters into the analysis settler (6),
- a step of combining said separated phases,
- a step of injecting said separated phases into said circuit (4) upstream of the outlet (22A, 22B, 22C) of the main settler (2).

14. Method for characterizing a multiphasic fluid according to the preceding claim, **characterized in that** it comprises a step of adjusting the flow rate of said analysis flow circulating through said analysis settler (6), by modifying the quantity of fluid forming said fraction.

15. Method for characterizing a multiphasic fluid according to the preceding claim, **characterized in that** it comprises a step of measuring, in at least one of said separated phases, the possibly present residual quantity of another phase of said fluid, and the following successive steps:
- a step of adjusting the analysis flow at a first measurement flow rate,
- a step of measuring in one of said separated phases the possibly present residual quantity of another phase of said fluid, allowing the determination of a measurement value corresponding to said first measurement flow rate,
- a step of storing said measurement value as a function of said measurement flow rate,
said flow adjustment, measurement and storage steps being repeated at different measurement flow rates so as to characterize the separation of said fluid as a function of the measurement flow rate.

16. Method for characterizing a multiphasic fluid according to one of claims 13 to 15, **characterized in that** said method comprises a step of locating, within said analysis settler (6), phases of said multiphasic fluid and/or a step of measuring the flow rate of one of said separated phases.
